# EUROPÄISCHE PATENTANMELDUNG

(11) **EP 4 799 656 A1**
(43) Veröffentlichungstag der Anmeldung: **02.09.2026**
(21) Anmeldenummer: 26158274.6
(22) Anmeldetag: 12.02.2026
(51) Int. Cl.: A61L 2/208, A61L 2/22, A61L 2/24, A61L 9/03

(54) **VERFAHREN ZUR REDUKTION DER KEIMDICHTE UND ENTSPRECHENDES GERÄT**

(30) Priorität: 27.02.2025 DE 102025107488
(71) Anmelder: Thermo Electron LED GmbH, 63505 Langenselbold (DE)
(72) Erfinder: Pieczarek, Waldemar, 63505 Langenselbold (DE); Ruckstuhl, Verena, 63628 Bad Soden - Salmünster (DE); D'Onofrio, Jennifer, 63505 Langenselbold (DE); Srinivasan, Kannan, Chelmsford, 01824 (US)
(74) Vertreter: Stellbrink & Partner Patentanwälte mbB

(57) **Zusammenfassung**

Die Erfindung betrifft ein Verfahren zum Reduzieren einer Keimdichte im Nutzraumes eines Geräts, umfassend Aufheizen des Nutzraums auf eine Prozesstemperatur, sodass eine relative Feuchte im Nutzraum unter einem oberen Feuchtegrenzwert liegt; Regelung der relativen Feuchte im Nutzraum auf einen ersten Zielwert für eine erste Dauer, wobei ein Befeuchtungssystem einen Wirkstoff einbringt; Deaktivieren der Regelung der relativen Feuchte; und Abbauen des Wirkstoffs im Nutzraum für eine Abbaudauer. Die Erfindung betrifft auch ein entsprechendes Gerät und ein entsprechendes System.

## Beschreibung

Die vorliegende Erfindung betrifft das Gebiet der Reduktion der Keimbelastung in Nutzräumen in Geräten, z.B. Labor-, Klima- und Wärmegeräten, und insbesondere der Sterilisation der Nutzräume.

Die Nutzräume von Geräten, insbesondere Labor-, Klima- und Wärmegeräten müssen für bestimmte Verwendungen möglichst steril sein, z.B. für Zellkultur- und Zelltherapieanwendungen. Ebenso gibt es Gegenstände, z.B. bestimmte chirurgische Geräte oder allgemeiner bestimmte Medizinprodukte, die einmalig oder regelmäßig sterilisiert werden müssen. Daher ist grundsätzlich eine Möglichkeit zur Reduktion der Keimdichte und vorzugsweise Sterilisation des Nutzraumes entsprechender Geräte erforderlich, um Verunreinigungen durch Bakterien, Viren, Schimmel oder andere unerwünschte Organismen im Nutzraum und/oder darin enthaltenen Gegenständen zu reduzieren bzw. vorteilhaft zu beseitigen.

Eine allgemein bekanntes und angewandtes Verfahren ist die Reduktion der Keimdichte durch Temperaturerhöhung. Dies kann jedoch mehrere Nachteile aufweisen. Zum einen kann dies ein langwieriger und energieaufwendiger Prozess sein, zum anderen gibt es Materialien, die entsprechend hohen Temperaturen nicht ausgesetzt werden können, ohne dabei Schaden zu nehmen.

Als Alternative sind Sterilisationsverfahren, die ein Sterilisationsmittel verwenden, bekannt. Aufgrund seiner chemischen Eigenschaften kommt hierfür insbesondere auch Wasserstoffperoxid (H₂O₂) in Frage. Wasserstoffperoxid wirkt jedoch stark ätzend und zytotoxisch, daher ist bei dem Umgang mit diesem Sterilisationsmittel darauf zu achten, dass dieses nicht freigesetzt wird, um Schäden für die Nutzer und die Umwelt zu vermeiden.

Beispielsweise sind Wasserstoffperoxid-Sterilisationskammern für Medizinprodukte bekannt, bei denen hoch konzentriertes Wasserstoffperoxid verwendet wird, z.B. in Vakuum oder in einem Plasma, und anschließend unschädlich gemacht wird. Dies sind jedoch reine Sterilisationskammern und keine Labor-, Klima- oder Wärmegeräte, wie z.B. Inkubatoren. Zusätzlich weisen diese Sterilisationskammern typischerweise ein geringes Volumen auf.

Ebenso sind mobile Generatoren bekannt, die zum Beispiel zur Dekontamination von Räumen, Isolatoren oder Oberflächen eingesetzt werden können und das Wasserstoffperoxid typischerweise verdampfen oder vernebeln. Diese Generatoren weisen jedoch typsicherweise keine geregelten Prozesse auf und sind daher nicht für wechselnde Umgebungsbedingungen geeignet. Zudem sind entsprechende Generatoren für die Dekontamination ganzer Laborräume ausgelegt und mit hohen Kosten verbunden.

Zusätzlich sind Sterilisationsgeräte zum Einbau in entsprechende Labor-, Klima- oder Wärmegeräte bekannt. Beispielsweise ist aus der US 2022/0243166 A1 eine Zerstäubungsvorrichtung bekannt, die mittels einer Membran eine Wasserstoffperoxid-Lösung zerstäubt. Hierbei können jedoch für den Nutzer gefährliche Rückstände im Nutzraum zurückbleiben und das entsprechende Gerät muss ein- und ausgebaut werden.

Der ReCO₂ver^{™} Inkubator der Firma Baker bietet die Möglichkeit den internen Vernebler zur Dekontamination des Inkubator-Innenraums zu verwenden. Hierzu wärmt ein Nutzer zunächst eine Wasserstoffperoxid-Lösung vor und füllt diese anschließend manuell in das Reservoir des Verneblers. Anschließend wird der Innenraum auf 45°C erhöht bevor der Vernebler für rund 20 Minuten läuft. Anschließend wird für ca. 130 Minuten ein UV-Licht eingeschaltet um suspendiertes Wasserstoffperoxid zu zersetzen. Anschließend leert der Nutzer das Reservoir manuell und reinigt dieses. Dieses Verfahren erfordert nachteilig das der Nutzer die Wasserstoffperoxid-Lösung manuell einfüllen, entfernen und entsorgen muss, sowie die anschließende Reinigung des Reservoirs. Dabei besteht die Gefahr, dass der Nutzer unmittelbar mit der Wasserstoffperoxid-Lösung in Kontakt kommt. Auch ist die Verneblung der Wasserstoffperoxid-Lösung nicht geregelt, sondern lediglich über die Dauer gesteuert.

Vor diesem Hintergrund ist es eine Aufgabe der vorliegenden Erfindung, die Unzulänglichkeiten und Nachteile des Standes der Technik zu überwinden oder zumindest zu reduzieren. Allgemein kann es Aufgabe der vorliegenden Erfindung sein, ein Gerät mit integrierter Möglichkeit zur Reduktion der Keimdichte und vorzugsweise Sterilisation des Nutzraumes bereitzustellen.

Die Aufgabe wird durch die Gegenstände der unabhängigen Patentansprüche gelöst. Vorteilhafte Weiterbildungen der Erfindung sind durch die abhängigen Patentansprüche, die folgende Beschreibung sowie die Figuren beschrieben.

Gemäß einem ersten Aspekt betrifft die Erfindung ein Verfahren zum Reduzieren einer Keimdichte im Nutzraumes eines Geräts, umfassend Aufheizen des Nutzraums auf eine Prozesstemperatur, sodass eine relative Feuchte im Nutzraum unter einem oberen Feuchtegrenzwert liegt; Regelung der relativen Feuchte im Nutzraum auf einen ersten Zielwert für eine erste Dauer, wobei ein Befeuchtungssystem einen Wirkstoff einbringt; Deaktivieren der Regelung der relativen Feuchte; und Abbauen des Wirkstoffs im Nutzraum für eine Abbaudauer.

D.h. die relative Feuchte im Nutzraum wird auf einen ersten Zielwert für eine erste Dauer geregelt, während ein Befeuchtungssystem einen Wirkstoff einbringt, beispielsweise verdampft oder vernebelt (insbesondere zerstäubt).

Es wird also ein Wirkstoff in den Nutzraum eingebracht. Dieser Wirkstoff kann die Keimdichte reduzieren. Beispielsweise kann es sich bei dem Wirkstoff um Wasserstoffperoxid H₂0₂ handeln. Während des Einbringens des Wirkstoffs wird die relative Feuchte im Nutzraum auf einen ersten Zielwert geregelt. Später wird der Wirkstoff im Nutzraum für eine Abbaudauer abgebaut.

Hierüber kann eine geeignete Reduzierung der Keimdichte, also beispielsweise eine geeignete Sterilisierung erzielt werden. Insbesondere erlaubt es die Erfindung, eine geeignete Reduzierung der Keimdichte mit relativ wenigen apparativen Anpassungen zu erzielen: Insbesondere wird für das Einbringen des Wirkstoffs ein Befeuchtungssystem verwendet, also ein System, das in vielen Geräten (beispielsweise in Inkubatoren) ohnehin vorhanden ist. Durch erfindungsgemäße Ausführungsformen kann insbesondere auch erreicht werden, dass die Reduzierung der Keimdichte mit relativ geringen Temperaturen (beispielsweise 55 °C oder geringer) erzielt wird. Das kann aus unterschiedlichen Gründen vorteilhaft sein: Das kann die Dauer des Verfahrens verkürzen, die benötigte Energie kann relativ gering sein und es bestehen geringere Anforderungen an die Materialien als dies bei höheren Temperaturen (beispielsweise von 180 °C) der Fall sein würde.

In Ausführungsformen der Erfindung kann vorgesehen sein, dass das Verfahren weiterhin umfasst: Einsetzen eines Wirkstoffbehälters, der eine Wirkstoffflüssigkeit enthält, und Einsetzen eines Abfallbehälters zur Aufnahme von Abfallflüssigkeiten.

In Ausführungsformen der Erfindung kann vorgesehen sein, dass das Verfahren weiterhin umfasst: Einbringen einer Abbaueinheit in den Nutzraum des Gerätes.

In Ausführungsformen der Erfindung kann vorgesehen sein, dass die Abbaueinheit vor dem Aufheizen in den Nutzraum eingebracht wird.

In Ausführungsformen der Erfindung kann vorgesehen sein, dass das Einbringen der Abbaueinheit in den Nutzraum das elektrische Verbinden der Abbaueinheit mit dem Gerät umfasst.

In Ausführungsformen der Erfindung kann vorgesehen sein, dass die Abbaueinheit dazu ausgelegt ist, den Wirkstoff zu zersetzen.

In Ausführungsformen der Erfindung kann vorgesehen sein, dass das Aufheizen des Nutzraumes auf die Prozesstemperatur ein Erhöhen der Prozesstemperatur umfasst, bis die relative Feuchte bei der Prozesstemperatur unter dem oberen Feuchtegrenzwert liegt.

In Ausführungsformen der Erfindung kann vorgesehen sein, dass das Aufheizen des Nutzraumes auf die Prozesstemperatur das Aufheizen des Nutzraumes auf einen Initialwert für die Prozesstemperatur umfasst, wobei der Initialwert durch den Nutzer bereitgestellt wird.

In Ausführungsformen der Erfindung kann vorgesehen sein, dass der Initialwert mindestens 37°C beträgt.

In Ausführungsformen der Erfindung kann vorgesehen sein, dass während und/oder nach dem Aufheizen auf den Initialwert die relative Feuchte im Nutzraum bestimmt und mit dem oberen Feuchtegrenzwert verglichen wird.

In Ausführungsformen der Erfindung kann vorgesehen sein, dass, falls die relative Feuchte den oberen Feuchtegrenzwert nicht unterschreitet, die Prozesstemperatur erhöht wird bis die relative Feuchte den oberen Feuchtegrenzwert unterschreitet.

In Ausführungsformen der Erfindung kann vorgesehen sein, dass das Erhöhen der Prozesstemperatur stufenweise ausgeführt wird, bis der obere Feuchtegrenzwert unterschritten wird.

In Ausführungsformen der Erfindung kann vorgesehen sein, dass das Erhöhen der Prozesstemperatur ein Bestimmen der Prozesstemperatur ausgehend von einer aktuellen relativen Feuchte und Temperatur anhand der Dampfdruckkurve umfasst.

In Ausführungsformen der Erfindung kann vorgesehen sein, dass die Prozesstemperatur größer oder gleich dem Initialwert ist und so gewählt wird, dass die relative Feuchte bei der Prozesstemperatur den oberen Feuchtegrenzwert unterschreitet.

In Ausführungsformen der Erfindung kann vorgesehen sein, dass die Prozesstemperatur mindestens 37°C beträgt.

In Ausführungsformen der Erfindung kann vorgesehen sein, dass die Prozesstemperatur maximal 75°C, vorzugsweise maximal 65°C, weiter vorzugsweise maximal 55°C beträgt.

In Ausführungsformen der Erfindung kann vorgesehen sein, dass die relative Feuchte während dem Aufheizen nicht geregelt wird.

In Ausführungsformen der Erfindung kann vorgesehen sein, dass der obere Feuchtegrenzwert im Bereich von 45% bis 75% relativer Feuchte, vorzugsweise im Bereich von 45% bis 60% relativer Feuchte, weiter bevorzugt im Bereich von 45% bis 55% relativer Feuchte liegt.

In Ausführungsformen der Erfindung kann vorgesehen sein, dass die Prozesstemperatur nach dem Aufheizen mindestens bis zum Abbauen des Wirkstoffs im Nutzraum konstant gehalten wird.

In Ausführungsformen der Erfindung kann vorgesehen sein, dass das Verfahren weiter umfasst: Erhöhung der relativen Feuchte im Nutzraum auf einen Wert oberhalb eines unteren Feuchtegrenzwertes.

In Ausführungsformen der Erfindung kann vorgesehen sein, dass die Erhöhung der relativen Feuchte im Nutzraum auf einen Wert oberhalb eines unteren Feuchtegrenzwertes vor der Regelung der relativen Feuchte auf einen ersten Zielwert für eine erste Dauer durchgeführt wird.

In Ausführungsformen der Erfindung kann vorgesehen sein, dass die Erhöhung der relativen Feuchte im Nutzraum auf einen Wert oberhalb eines unteren Feuchtegrenzwertes nach dem Aufheizen des Nutzraums auf die Prozesstemperatur durchgeführt wird.

In Ausführungsformen der Erfindung kann vorgesehen sein, dass die Erhöhung der relativen Feuchte im Nutzraum auf einen Wert oberhalb eines unteren Feuchtegrenzwertes das Verdampfen von Wasser umfasst.

In Ausführungsformen der Erfindung kann vorgesehen sein, dass der untere Feuchtegrenzwert im Bereich von 30% bis 40% relativer Feuchte liegt.

In Ausführungsformen der Erfindung kann vorgesehen sein, dass die Erhöhung der relativen Feuchte im Nutzraum auf einen Wert oberhalb eines unteren Feuchtegrenzwertes vor der Regelung der relativen Feuchte im Nutzraum auf einen ersten Zielwert durchgeführt wird.

In Ausführungsformen der Erfindung kann vorgesehen sein, dass der erste Zielwert einen ersten Betrag über der Feuchte zu Beginn der Regelung der relativen Feuchte auf einen ersten Zielwert liegt.

In Ausführungsformen der Erfindung kann vorgesehen sein, dass der erste Betrag im Bereich von 10%-40% relativer Feuchte, weiter bevorzugt im Bereich von 15%-30% relativer Feuchte, noch weiter Bevorzugt im Bereich von 15%-25% relativer Feuchte liegt.

In Ausführungsformen der Erfindung kann vorgesehen sein, dass die erste Dauer im Bereich von 5 min bis 120 min, vorzugsweise im Bereich von 30 min bis 90 min, weiter bevorzugt im Bereich von 40 bis 70 min liegt.

In Ausführungsformen der Erfindung kann vorgesehen sein, dass das Einbringen des Wirkstoffs das Verdampfen oder Vernebeln einer Wirkstoffflüssigkeit im Befeuchter des Befeuchtungssystems umfasst.

In Ausführungsformen der Erfindung kann vorgesehen sein, dass die Wirkstoffflüssigkeit in einem Wirkstoffbehälter und/oder an einem Wirkstoffanschluss des Gerätes bereitgestellt wird.

In Ausführungsformen der Erfindung kann vorgesehen sein, dass die Wirkstoffflüssigkeit eine Wirkstofflösung ist.

In Ausführungsformen der Erfindung kann vorgesehen sein, dass der Wirkstoff ein Sterilisationsmittel ist.

In Ausführungsformen der Erfindung kann vorgesehen sein, dass der Wirkstoff Wasserstoffperoxid (H₂O₂) ist.

In Ausführungsformen der Erfindung kann vorgesehen sein, dass das Verfahren weiterhin umfasst: nach Ablauf der ersten Dauer, Bestimmen der aktuellen relativen Feuchte im Nutzraum und Regelung der relativen Feuchte auf einen zweiten Zielwert für eine zweite Dauer, wobei das Befeuchtungssystem eine Wirkstoffflüssigkeit einbringt.

In Ausführungsformen der Erfindung kann vorgesehen sein, dass der zweite Zielwert einen zweiten Betrag über der aktuellen relativen Feuchte liegt.

In Ausführungsformen der Erfindung kann vorgesehen sein, dass der zweite Betrag im Bereich von 10%-40% relativer Feuchte, weiter bevorzugt im Bereich von 15%-30% relativer Feuchte, noch weiter Bevorzugt im Bereich von 15%-25% relativer Feuchte liegt.

In Ausführungsformen der Erfindung kann vorgesehen sein, dass die zweite Dauer im Bereich von 5 min bis 120 min, vorzugsweise im Bereich von 30 min bis 90 min, weiter bevorzugt im Bereich von 40 bis 70 min liegt.

In Ausführungsformen der Erfindung kann vorgesehen sein, dass die Schritte Deaktivieren der Regelung, Abbauen des Wirkstoffs und Spülen erst nach Ablauf der ersten und/oder zweiten Dauer erfolgen.

In Ausführungsformen der Erfindung kann vorgesehen sein, dass die Regelung der relativen Feuchte im Nutzraum auf einen ersten Zielwert und/oder einen zweiten Zielwert das aktive Verteilen der Luft im Nutzraum des Gerätes umfasst.

In Ausführungsformen der Erfindung kann vorgesehen sein, dass das Verfahren das Verdünnen der Wirkstoffflüssigkeit vor dem Verdampfen oder Vernebeln umfasst.

In Ausführungsformen der Erfindung kann vorgesehen sein, dass das Abbauen des Wirkstoffs im Nutzraum das Aktivieren der Neutralisierungseinheit umfasst.

In Ausführungsformen der Erfindung kann vorgesehen sein, dass das Abbauen des Wirkstoffs ein Zersetzen des Wirkstoffs umfasst.

In Ausführungsformen der Erfindung kann vorgesehen sein, dass die Abbaudauer so gewählt ist, dass eine Restkonzentration des gasförmigen Wirkstoffs in dem Nutzraum keine Gefahr für einen Nutzer darstellt.

In Ausführungsformen der Erfindung kann vorgesehen sein, dass die Abbaudauer in einem Bereich von 10 min bis 600 min, vorzugsweise in einem Bereich von 20 min bis 400 min, weiter vorzugsweise in einem Bereich von 30 min bis 240 min liegt.

In Ausführungsformen der Erfindung kann vorgesehen sein, dass das Verfahren weiter umfasst: Spülen zumindest eines Teils von Zuleitungen zu einem Befeuchter des Befeuchtungssystems.

In Ausführungsformen der Erfindung kann vorgesehen sein, dass das Spülen bidirektional erfolgt.

In Ausführungsformen der Erfindung kann vorgesehen sein, dass das Spülen das Entleeren von Flüssigkeitsleitungen in einen Abfallbehälter umfasst.

In Ausführungsformen der Erfindung kann vorgesehen sein, dass das Spülen das Füllen der entleerten Flüssigkeitsleitungen mit Frischwasser umfasst.

In Ausführungsformen der Erfindung kann vorgesehen sein, dass erneut das Spülen das Entleeren der Flüssigkeitsleitungen in den Abfallbehälter umfasst.

In Ausführungsformen der Erfindung kann vorgesehen sein, dass das Entleeren und/oder Füllen mittels einer bidirektionalen Pumpe erfolgt.

In Ausführungsformen der Erfindung kann vorgesehen sein, dass das Verfahren weiterhin umfasst: Verriegeln einer Tür zum Nutzraum des Geräts, wobei das Verriegeln vor dem Aufheizen des Nutzraums auf eine Prozesstemperatur durchgeführt wird.

In Ausführungsformen der Erfindung kann vorgesehen sein, dass das Verfahren weiterhin umfasst: Durchführen eines automatischen Checks des Geräts, wobei der Check vor dem Aufheizen des Nutzraums auf eine Prozesstemperatur durchgeführt wird und das Aufheizen des Nutzraums auf eine Prozesstemperatur nur durchgeführt wird, wenn der Check erfolgreich ist.

In Ausführungsformen der Erfindung kann vorgesehen sein, dass das Durchführen des automatischen Checks umfasst:
- Checken eines Temperiersystems,
- Checken eines Feuchtesystems,
- Checken eines Lüftungssystems, und/oder
- Checken einer Wassermenge in einem Vorratsbehälter.

In Ausführungsformen der Erfindung kann vorgesehen sein, dass das Durchführen des automatischen Checks umfasst: - Checken eines Status der Abbaueinheit.

In Ausführungsformen der Erfindung kann vorgesehen sein, dass das Befeuchtungssystem einen Flüssigkeitssensor umfasst, wobei das Durchführen des automatischen Checks umfasst: - Checken eines Status des Flüssigkeitssensors.

In Ausführungsformen der Erfindung kann vorgesehen sein, dass mindestens während der ersten Zeitdauer ein Alarmsystem aktiv ist, das mindestens eine Funktion des Geräts überwacht.

Insgesamt können damit unterschiedliche Sicherheitsmechanismen vorgesehen sein. Die Tür kann verriegelt werden. Weiter kann vorgesehen sein, dass der Prozess nur gestartet werden kann, wenn im Temperatur-, Feuchte- und Lüftungssystem kein Fehler vorliegt und der Wasservorratsbehälter mit einer ausreichend großen Wassermenge gefüllt ist. Zusätzlich kann geprüft werden, ob die Abbaueinheit (die auch als Neutralisationseinheit bezeichnet werden kann) angeschlossen ist. Falls ein Sensor zur Detektion der Wassersäule (beispielsweise in einem Schlauch vor dem Verdampfer) eingesetzt wird, kann dieser ebenfalls auf Funktion geprüft werden. Während der Einbringung des Wirkstoffs (was beispielsweise auch als Begasung bezeichnet werden kann) kann auch ständig das gleiche Alarmsystem aktiv sein, das in einem möglichen Inkubationsbetrieb verwendet wird. Es kann unterschiedliche Funktionen, wie z.B.: Ausfall von Sensoren, des Lüfters, Ausfall von Heizungen, Überwachung des Wasservorrats, und/oder Überschreitung von Sollwerten der Regler.

In Ausführungsformen der Erfindung kann vorgesehen sein, dass das Gerät ein Laborgerät ist.

In Ausführungsformen der Erfindung kann vorgesehen sein, dass das Gerät ein Klima- oder Wärmegerät ist.

In Ausführungsformen der Erfindung kann vorgesehen sein, dass das Gerät ein Inkubator ist, beispielsweise ein CO₂-Inkubator und/oder ein Reach-In-Inkubator.

Nach einem weiteren Aspekt ist die Erfindung auf ein Gerät gerichtet. Das Gerät umfasst: einen Nutzraum; ein aktives Befeuchtungssystem, das ausgelegt ist, eine relative Luftfeuchte im Nutzraum zu erhöhen; einen Feuchtesensor zum Messen der relativen Feuchte im Nutzraum; ein Temperiersystem, das ausgelegt ist, eine Temperatur im Nutzraum zu erhöhen; einen Temperatursensor zum Messen der Temperatur im Nutzraum; wobei das Befeuchtungssystem mindestens einen Wirkstoffanschluss umfasst, der zur Verbindung mit einem Wirkstoffbehälter, der eine Wirkstoffflüssigkeit mit einem Wirkstoff enthält, ausgelegt ist; und wobei das Gerät dazu ausgelegt ist, den Wirkstoff über das Befeuchtungssystem in den Nutzraum einzubringen.

In Ausführungsformen der Erfindung kann vorgesehen sein, dass das Befeuchtungssystem weiterhin mindestens einen Abfallanschluss umfasst, der zur Verbindung mit einem Abfallbehälter ausgelegt ist wobei das Gerät dazu ausgelegt ist, zumindest einen Teil des Befeuchtungssystems zu spülen und dabei anfallende Abfallflüssigkeit zu dem Abfallanschluss zu leiten.

In Ausführungsformen der Erfindung kann vorgesehen sein, dass das Befeuchtungssystem einen Befeuchter umfasst, der dazu ausgelegt ist Flüssigkeiten zu verdampfen und/oder zu vernebeln.

In Ausführungsformen der Erfindung kann vorgesehen sein, dass der Befeuchter ein Verdampfer und/oder ein Vernebler ist.

In Ausführungsformen der Erfindung kann vorgesehen sein, dass das Befeuchtungssystem einen Wasseranschluss umfasst, der zur Verbindung mit einem Wasserreservoir, welches Frischwasser bereitstellt, ausgelegt ist.

In Ausführungsformen der Erfindung kann vorgesehen sein, dass das Befeuchtungssystem eine Pumpe umfasst.

In Ausführungsformen der Erfindung kann vorgesehen sein, dass die Pumpe eine bidirektionale Pumpe ist.

Es versteht sich, dass eine bidirektionale Pumpe in beide Richtungen fördern kann.

In Ausführungsformen der Erfindung kann vorgesehen sein, dass die Pumpe dazu ausgelegt ist Flüssigkeiten vom Wirkstoffanschluss zum Befeuchter zu fördern.

In Ausführungsformen der Erfindung kann vorgesehen sein, dass die Pumpe dazu ausgelegt ist Flüssigkeiten vom Wasseranschluss zum Befeuchter zu fördern.

In Ausführungsformen der Erfindung kann vorgesehen sein, dass das Befeuchtungssystem eine Ventilmatrix umfasst, die dazu ausgelegt ist fluidische Verbindungen zwischen verschiedenen Anschlüssen zu ermöglichen.

In Ausführungsformen der Erfindung kann vorgesehen sein, dass die Ventilmatrix weiter dazu ausgelegt ist die Durchflussmenge für mindestens eine fluidische Verbindung einzustellen.

In Ausführungsformen der Erfindung kann vorgesehen sein, dass die Ventilmatrix zwischen der Pumpe und dem Wirkstoffanschluss angeordnet ist.

In Ausführungsformen der Erfindung kann vorgesehen sein, dass die Ventilmatrix zwischen der Pumpe und dem Wasseranschluss angeordnet ist.

In Ausführungsformen der Erfindung kann vorgesehen sein, dass die Ventilmatrix zwischen der Pumpe und dem Abfallanschluss angeordnet ist.

In Ausführungsformen der Erfindung kann vorgesehen sein, dass die Ventilmatrix mindestens 2 Ventile umfasst.

In Ausführungsformen der Erfindung kann vorgesehen sein, dass das Gerät zusätzlich eine Luftverteilung umfasst, die dazu ausgelegt ist die Luft im Nutzraum umzuwälzen.

In Ausführungsformen der Erfindung kann vorgesehen sein, dass die Luftverteilung einen Lüfter umfasst.

In Ausführungsformen der Erfindung kann vorgesehen sein, dass das Gerät weiter ein Belüftungssystem umfasst, das dazu ausgelegt ist, dem Nutzraum Zuluft zuzuführen.

In Ausführungsformen der Erfindung kann vorgesehen sein, dass das Gerät dazu ausgelegt ist das beschriebene Verfahren durchzuführen.

In Ausführungsformen der Erfindung kann vorgesehen sein, dass das Gerät eine Verarbeitungseinheit umfasst, die dazu ausgelegt ist das Gerät zu steuern.

In Ausführungsformen der Erfindung kann vorgesehen sein, dass die Verarbeitungseinheit dazu ausgelegt ist das beschriebene Verfahren durchzuführen.

In Ausführungsformen der Erfindung kann vorgesehen sein, dass die Wirkstoffflüssigkeit eine Wirkstofflösung ist.

In Ausführungsformen der Erfindung kann vorgesehen sein, dass der Wirkstoff ein Sterilisationsmittel ist.

In Ausführungsformen der Erfindung kann vorgesehen sein, dass der Wirkstoff Wasserstoffperoxid (H₂O₂) ist.

In Ausführungsformen der Erfindung kann vorgesehen sein, dass das Gerät ein Laborgerät ist.

In Ausführungsformen der Erfindung kann vorgesehen sein, dass das Gerät ein Klima- oder Wärmegerät ist.

In Ausführungsformen der Erfindung kann vorgesehen sein, dass das Gerät ein Inkubator ist, beispielsweise ein CO₂-Inkubator und/oder ein Reach-In-Inkubator.

In Ausführungsformen der Erfindung kann vorgesehen sein, dass das Befeuchtungssystem weiter einen Flüssigkeitssensor umfasst, der zwischen dem Befeuchter und der Pumpe angeordnet ist.

In Ausführungsformen der Erfindung kann vorgesehen sein, dass das Gerät ein Gerät wie vorstehend beschrieben ist.

Gemäß einem weiteren Aspekt betrifft die Erfindung ein System. Das System umfasst das beschriebene Gerät und eine Abbaueinheit, die dazu ausgelegt ist, einen Wirkstoff im Nutzraum des Gerätes abzubauen.

In Ausführungsformen der Erfindung kann vorgesehen sein, dass die Abbaueinheit dazu ausgelegt ist im Nutzraum platziert und elektrisch mit dem Gerät verbunden zu werden.

In Ausführungsformen der Erfindung kann vorgesehen sein, dass die Abbaueinheit eine UV-Lampe umfasst.

In Ausführungsformen der Erfindung kann vorgesehen sein, dass die Abbaueinheit einen Reaktionspartner und/oder Katalysator für den Wirkstoff umfasst.

In Ausführungsformen der Erfindung kann vorgesehen sein, dass die Abbaueinheit einen Lüfter umfasst, der dazu ausgelegt ist Luft über den Reaktionspartner und/oder Katalysator zu leiten.

Allgemein sind Ausführungsformen der Erfindung also auf ein Verfahren, ein Gerät und ein System zur Reduzierung der Keimdichte gerichtet.

Es ist verständlich, dass in einem Standard-Gerät (beispielsweise in einem Inkubator) mit aktivem Befeuchtungssystem üblicherweise die folgenden Komponenten vorhanden sind
- Wasserbehälter oder externer Wasseranschluss
- Dampferzeuger
- Rohr oder Schlauchsystem für Hydraulikverbindungen
- Pumpe oder Ventil zur Dosierung des Wassers
- Feuchtesensor
- Luftverteilung im Nutzraum
- Heizung(en) und
- Temperatursensor.

Nach Ausführungsformen der vorliegenden Erfindung kann ein solches Gerät bzw. ein entsprechendes System mit den vorhandenen Komponenten für andere Aufgaben erweitert werden. Das System kann so erweitert werden, dass zu dem angeschlossenen Wasserbehälter noch andere Behälter mit weiteren flüssigen Medien angeschlossen werden können. Über Ventile kann gewählt werden, aus welchem Behälter die Flüssigkeit zum Verdampfer dosiert wird. Nach einer Verdampfung aus diesen anderen Behältern, können eventuelle Rückstände in der Hydraulikanlage mit Wasser aus dem 1. Behälter ausgespült werden. Das Wasserbehälter 1 bleibt am System angeschlossen.

Die Restflüssigkeiten im Hydrauliksystem und Spülflüssigkeit kann in einen 3. Behälter als Abfallflüssigkeit geleitet werden.

Ausführungsformen der Erfindung umfassen also die Erweiterung des Befeuchtungssystems in ein Sterilisationssystem für den Innenraum.

Folgende Aufgaben können durch erfindungsgemäße Ausführungsformen System und die Verdampfung von entsprechenden Sterilisationsmitteln erfüllt werden
- Reduktion der Keimbelastung im Nutzraum,
- Desinfektion des Nutzraums, und/oder
- Sterilisation des Nutzraums.

Zusätzlich kann eine Ventileinheit angeschlossen werden, um ein anderes Sterilisationsmittel aus verschiedenen Ausgangschemikalien zu mischen oder um aus einem Konzentrat eine Verdünnungslösung herzustellen.

Mittels erfindungsgemäßen Ausführungsformen können die folgenden Vorteile realisiert werden:
Dieselben und bereits vorhandenen Bauteile, die die Standard-Befeuchtungsfunktion realisieren, können genutzt werden. Es sind also relativ wenige weitere Anpassungen an den Bauteilen notwendig.

Es kann ein automatisierter und geregelter Prozess realisiert werden und eine Regelung über das Gerät (beispielsweise den Inkubator) erfolgen.

Der Prozess kann überprüft werden und es kann eine automatische Anpassung des Prozesses an die Umgebungsbedingungen am Aufstellungsort erreicht werden (Abhängig von der rel. Feuchte wird die Sterilisationstemperatur angepasst).

Zumindest ein Teil des Leitungssystem für Wasser kann mit flüssigem Sterilisationsmittel dekontaminiert werden.

Der Prozess kann bei 37°C oder geringfügig höheren Temperaturen (beispielsweise 50°C) ablaufen, sodass keine Abkühlzeit (oder nur eine relativ geringe Abkühlzeit) notwendig ist.

Allgemein kann der Prozess auch schneller sein als Sterilisationsroutinen, die beispielsweise bei 180°C durchgeführt werden.

Eine Isolationsdicke des Geräts kann reduziert werden im Vergleich zur Hitze-Sterilisation (beispielsweise bei 180°C).

Die Materialauswahl für das Gesamtsystem kann erweitert werden, da 180°C Beständigkeit sehr hohe Anforderungen hat, was bei Ausführungsformen der Erfindung nicht notwendig ist.

Es ist verständlich, dass es möglich ist, geringere Oberflächentemperaturen am Gerät (z.B. Inkubator) während des Prozesses zu haben als bei Sterilisationen mit ca. 180°C.

Insgesamt kann auch eine bessere Eignung gegeben sein für Reinraumanwendungen als Prozesse bei 180°C (kein Risiko zur vermehrten Freisetzung von Partikel und Störung der Reinraumluftströmung durch thermische Effekte).

Die Erfindung wird auch durch die folgenden nummerierten Ausführungsformen definiert.

Nachfolgend wird auf Verfahrensausführungsformen Bezug genommen. Diese Ausführungsformen sind durch ein V, gefolgt von einer Zahl gekennzeichnet. Wenn im Folgenden von Verfahrensausführungsformen oder auch V-Ausführungsformen die Rede ist, sind diese Ausführungsformen gemeint.
V1. Verfahren zum Reduzieren einer Keimdichte im Nutzraumes eines Geräts, umfassend
   Aufheizen des Nutzraums auf eine Prozesstemperatur, sodass eine relative Feuchte im Nutzraum unter einem oberen Feuchtegrenzwert liegt;
   Regelung der relativen Feuchte im Nutzraum auf einen ersten Zielwert für eine erste Dauer, wobei ein Befeuchtungssystem einen Wirkstoff einbringt;
   Deaktivieren der Regelung der relativen Feuchte; und
   Abbauen des Wirkstoffs im Nutzraum für eine Abbaudauer.
D.h. die relative Feuchte im Nutzraum wird auf einen ersten Zielwert für eine erste Dauer geregelt, während ein Befeuchtungssystem einen Wirkstoff einbringt, beispielsweise verdampft oder vernebelt (insbesondere zerstäubt).
V2. Verfahren nach der vorstehenden Verfahrensausführungsform, wobei das Verfahren weiterhin umfasst
   Einsetzen eines Wirkstoffbehälters, der eine Wirkstoffflüssigkeit enthält, und
   Einsetzen eines Abfallbehälters zur Aufnahme von Abfallflüssigkeiten.
V3. Verfahren nach einer der vorstehenden Verfahrensausführungsformen, wobei das Verfahren weiterhin umfasst
   Einbringen einer Abbaueinheit in den Nutzraum des Gerätes.
V4. Verfahren nach der vorstehenden Verfahrensausführungsform, wobei die Abbaueinheit vor dem Aufheizen in den Nutzraum eingebracht wird.
V5. Verfahren nach einer der 2 vorstehenden Verfahrensausführungsformen, wobei das Einbringen der Abbaueinheit in den Nutzraum das elektrische Verbinden der Abbaueinheit mit dem Gerät umfasst.
V6. Verfahren nach einer der 3 vorstehenden Verfahrensausführungsformen, wobei die Abbaueinheit dazu ausgelegt ist, den Wirkstoff zu zersetzen.
V7. Verfahren nach einer der vorstehenden Verfahrensausführungsformen, wobei das Aufheizen des Nutzraumes auf die Prozesstemperatur ein Erhöhen der Prozesstemperatur umfasst, bis die relative Feuchte bei der Prozesstemperatur unter dem oberen Feuchtegrenzwert liegt.
V8. Verfahren nach einer der vorstehenden Verfahrensausführungsformen, wobei das Aufheizen des Nutzraumes auf die Prozesstemperatur das Aufheizen des Nutzraumes auf einen Initialwert für die Prozesstemperatur umfasst, wobei der Initialwert durch den Nutzer bereitgestellt wird.
V9. Verfahren nach der vorstehenden Verfahrensausführungsform, wobei der Initialwert mindestens 37°C beträgt.
V10. Verfahren nach einer der 2 vorstehenden Verfahrensausführungsformen, wobei während und/oder nach dem Aufheizen auf den Initialwert die relative Feuchte im Nutzraum bestimmt und mit dem oberen Feuchtegrenzwert verglichen wird.
V11. Verfahren nach der vorstehenden Verfahrensausführungsform, wobei, falls die relative Feuchte den oberen Feuchtegrenzwert nicht unterschreitet, die Prozesstemperatur erhöht wird bis die relative Feuchte den oberen Feuchtegrenzwert unterschreitet.
V12. Verfahren nach Verfahrensausführungsform V7 oder V11, wobei das Erhöhen der Prozesstemperatur stufenweise ausgeführt wird, bis der obere Feuchtegrenzwert unterschritten wird.
V13. Verfahren nach Verfahrensausführungsform V7 oder V11, wobei das Erhöhen der Prozesstemperatur ein Bestimmen der Prozesstemperatur ausgehend von einer aktuellen relativen Feuchte und Temperatur anhand der Dampfdruckkurve umfasst.
V14. Verfahren nach einer der vorstehenden Verfahrensausführungsformen, wobei die Prozesstemperatur größer oder gleich dem Initialwert ist und so gewählt wird, dass die relative Feuchte bei der Prozesstemperatur den oberen Feuchtegrenzwert unterschreitet.
V15. Verfahren nach einer der vorstehenden Verfahrensausführungsformen, wobei die Prozesstemperatur mindestens 37°C beträgt.
V16. Verfahren nach einer der vorstehenden Ausführungsformen, wobei die Prozesstemperatur maximal 75°C, vorzugsweise maximal 65°C, weiter vorzugsweise maximal 55°C beträgt.
V17. Verfahren nach einer der vorstehenden Verfahrensausführungsformen, wobei die relative Feuchte während dem Aufheizen nicht geregelt wird.
V18. Verfahren nach einer der Vorstehenden Verfahrensausführungsformen, wobei der obere Feuchtegrenzwert im Bereich von 45% bis 75% relativer Feuchte, vorzugsweise im Bereich von 45% bis 60% relativer Feuchte, weiter bevorzugt im Bereich von 45% bis 55% relativer Feuchte liegt.
V19. Verfahren nach einer der vorstehenden Verfahrensausführungsformen, wobei die Prozesstemperatur nach dem Aufheizen mindestens bis zum Abbauen des Wirkstoffs im Nutzraum konstant gehalten wird.
V20. Verfahren nach einer der vorstehenden Verfahrensausführungsformen, wobei das Verfahren weiter umfasst:
   Erhöhung der relativen Feuchte im Nutzraum auf einen Wert oberhalb eines unteren Feuchtegrenzwertes.
V21. Verfahren nach der vorstehenden Ausführungsform,
   wobei die Erhöhung der relativen Feuchte im Nutzraum auf einen Wert oberhalb eines unteren Feuchtegrenzwertes vor der Regelung der relativen Feuchte auf einen ersten Zielwert für eine erste Dauer durchgeführt wird.
V22. Verfahren nach einer der 2 vorstehenden Ausführungsformen,
   wobei die Erhöhung der relativen Feuchte im Nutzraum auf einen Wert oberhalb eines unteren Feuchtegrenzwertes nach dem Aufheizen des Nutzraums auf die Prozesstemperatur durchgeführt wird.
V23. Verfahren nach einer der 3 vorstehenden Ausführungsformen,
   wobei die Erhöhung der relativen Feuchte im Nutzraum auf einen Wert oberhalb eines unteren Feuchtegrenzwertes das Verdampfen von Wasser umfasst.
V24. Verfahren nach einer der 4 vorstehenden Ausführungsformen, wobei der untere Feuchtegrenzwert im Bereich von 30% bis 40% relativer Feuchte liegt.
V25. Verfahren nach einer der 5 vorstehenden Ausführungsformen, wobei die Erhöhung der relativen Feuchte im Nutzraum auf einen Wert oberhalb eines unteren Feuchtegrenzwertes vor der Regelung der relativen Feuchte im Nutzraum auf einen ersten Zielwert durchgeführt wird.
V26. Verfahren nach einer der vorstehenden Verfahrensausführungsformen, wobei der erste Zielwert einen ersten Betrag über der Feuchte zu Beginn der Regelung der relativen Feuchte auf einen ersten Zielwert liegt.
V27. Verfahren nach der vorstehenden Verfahrensausführungsform, wobei der erste Betrag im Bereich von 10%-40% relativer Feuchte, weiter bevorzugt im Bereich von 15%-30% relativer Feuchte, noch weiter Bevorzugt im Bereich von 15%-25% relativer Feuchte liegt.
V28. Verfahren nach einer der vorstehenden Verfahrensausführungsformen, wobei die erste Dauer im Bereich von 5 min bis 120 min, vorzugsweise im Bereich von 30 min bis 90 min, weiter bevorzugt im Bereich von 40 bis 70 min liegt.
V29. Verfahren nach einer der vorstehenden Verfahrensausführungsformen, wobei das Einbringen des Wirkstoffs das Verdampfen oder Vernebeln einer Wirkstoffflüssigkeit im Befeuchter des Befeuchtungssystems umfasst.
V30. Verfahren nach einer der vorstehenden Verfahrensausführungsformen, wobei die Wirkstoffflüssigkeit in einem Wirkstoffbehälter und/oder an einem Wirkstoffanschluss des Gerätes bereitgestellt wird.
V31. Verfahren nach einer der vorstehenden Verfahrensausführungsformen, wobei die Wirkstoffflüssigkeit eine Wirkstofflösung ist.
V32. Verfahren nach einer der vorstehenden Verfahrensausführungsformen, wobei der Wirkstoff ein Sterilisationsmittel ist.
V33. Verfahren nach einer der vorstehenden Verfahrensausführungsformen, wobei der Wirkstoff Wasserstoffperoxid (H₂O₂) ist.
V34. Verfahren nach einer der vorstehenden Verfahrensausführungsformen, wobei das Verfahren weiterhin umfasst:
   nach Ablauf der ersten Dauer,
   Bestimmen der aktuellen relativen Feuchte im Nutzraum und
   Regelung der relativen Feuchte auf einen zweiten Zielwert für eine zweite Dauer, wobei das Befeuchtungssystem eine Wirkstoffflüssigkeit einbringt.
V35. Verfahren nach der vorstehenden Verfahrensausführungsform, wobei der zweite Zielwert einen zweiten Betrag über der aktuellen relativen Feuchte liegt.
V36. Verfahren nach der vorstehenden Verfahrensausführungsform, wobei der zweite Betrag im Bereich von 10%-40% relativer Feuchte, weiter bevorzugt im Bereich von 15%-30% relativer Feuchte, noch weiter Bevorzugt im Bereich von 15%-25% relativer Feuchte liegt.
V37. Verfahren nach einer der 3 vorstehenden Verfahrensausführungsformen, wobei die zweite Dauer im Bereich von 5 min bis 120 min, vorzugsweise im Bereich von 30 min bis 90 min, weiter bevorzugt im Bereich von 40 bis 70 min liegt.
V38. Verfahren nach einer der vorstehenden Verfahrensausführungsformen, wobei die Schritte Deaktivieren der Regelung, Abbauen des Wirkstoffs und Spülen erst nach Ablauf der ersten und/oder zweiten Dauer erfolgen.
V39. Verfahren nach einer der vorstehenden Verfahrensausführungsformen, wobei die Regelung der relativen Feuchte im Nutzraum auf einen ersten Zielwert und/oder einen zweiten Zielwert das aktive Verteilen der Luft im Nutzraum des Gerätes umfasst.
V40. Verfahren nach einer der vorstehenden Verfahrensausführungsform und mit den Merkmalen von V29, wobei das Verfahren das Verdünnen der Wirkstoffflüssigkeit vor dem Verdampfen oder Vernebeln umfasst.
V41. Verfahren nach einer der vorstehenden Verfahrensausführungsformen und mit den Merkmalen von V3, wobei das Abbauen des Wirkstoffs im Nutzraum das Aktivieren der Neutralisierungseinheit umfasst.
V42. Verfahren nach einer der vorstehenden Verfahrensausführungsformen, wobei das Abbauen des Wirkstoffs ein Zersetzen des Wirkstoffs umfasst.
V43. Verfahren nach einer der vorstehenden Verfahrensausführungsformen, wobei die Abbaudauer so gewählt ist, dass eine Restkonzentration des gasförmigen Wirkstoffs in dem Nutzraum keine Gefahr für einen Nutzer darstellt.
V44. Verfahren nach einer der vorstehenden Verfahrensausführungsformen, wobei die Abbaudauer in einem Bereich von 10 min bis 600 min, vorzugsweise in einem Bereich von 20 min bis 400 min, weiter vorzugsweise in einem Bereich von 30 min bis 240 min liegt.
V45. Verfahren nach einer der vorstehenden Verfahrensausführungsformen, wobei das Verfahren weiter umfasst: Spülen zumindest eines Teils von Zuleitungen zu einem Befeuchter des Befeuchtungssystems.
V46. Verfahren nach der vorstehenden Ausführungsform, wobei das Spülen bidirektional erfolgt.
V47. Verfahren nach einer der 2 vorstehenden Verfahrensausführungsformen, wobei das Spülen das Entleeren von Flüssigkeitsleitungen in einen Abfallbehälter umfasst.
V48. Verfahren nach der vorstehenden Verfahrensausführungsform, wobei das Spülen das Füllen der entleerten Flüssigkeitsleitungen mit Frischwasser umfasst.
V49. Verfahren nach der vorstehenden Verfahrensausführungsform, wobei erneut das Spülen das Entleeren der Flüssigkeitsleitungen in den Abfallbehälter umfasst.
V50. Verfahren nach einer der 5 vorstehenden Verfahrensausführungsformen, wobei das Entleeren und/oder Füllen mittels einer bidirektionalen Pumpe erfolgt.
V51. Verfahren nach einer der vorstehenden Ausführungsformen, wobei das Verfahren weiterhin umfasst: Verriegeln einer Tür zum Nutzraum des Geräts, wobei das Verriegeln vor dem Aufheizen des Nutzraums auf eine Prozesstemperatur durchgeführt wird.
V52. Verfahren nach einer der vorstehenden Ausführungsformen, wobei das Verfahren weiterhin umfasst: Durchführen eines automatischen Checks des Geräts, wobei der Check vor dem Aufheizen des Nutzraums auf eine Prozesstemperatur durchgeführt wird und das Aufheizen des Nutzraums auf eine Prozesstemperatur nur durchgeführt wird, wenn der Check erfolgreich ist.
V53. Verfahren nach der vorstehenden Ausführungsform, wobei das Durchführen des automatischen Checks umfasst:
   - Checken eines Temperiersystems,
   - Checken eines Feuchtesystems,
   - Checken eines Lüftungssystems, und/oder
   - Checken einer Wassermenge in einem Vorratsbehälter.
V54. Verfahren nach einer der 2 vorstehenden Ausführungsformen mit den Merkmalen der Ausführungsform V3, wobei das Durchführen des automatischen Checks umfasst:
   - Checken eines Status der Abbaueinheit.
V55. Verfahren nach einer der 3 vorstehenden Ausführungsformen, wobei das Befeuchtungssystem einen Flüssigkeitssensor umfasst, wobei das Durchführen des automatischen Checks umfasst:
   - Checken eines Status des Flüssigkeitssensors.
V56. Verfahren nach einer der vorstehenden Ausführungsformen, wobei mindestens während der ersten Zeitdauer ein Alarmsystem aktiv ist, das mindestens eine Funktion des Geräts überwacht.
V57. Verfahren nach einer der vorstehenden Verfahrensausführungsformen, wobei das Gerät ein Laborgerät ist.
V58. Verfahren nach einer der vorstehenden Verfahrensausführungsformen, wobei das Gerät ein Klima- oder Wärmegerät ist.
V59. Verfahren nach einer der vorstehenden Verfahrensausführungsformen,, wobei das Gerät ein Inkubator ist.

Nachfolgend wird auf Geräteausführungsformen Bezug genommen. Diese Ausführungsformen sind durch ein G, gefolgt von einer Zahl gekennzeichnet. Wenn im Folgenden von Gerätausführungsformen oder auch G-Ausführungsformen die Rede ist, sind diese Ausführungsformen gemeint.
G1. Gerät umfassend
   einen Nutzraum;
   ein aktives Befeuchtungssystem, das ausgelegt ist, eine relative Feuchte im Nutzraum zu erhöhen;
   einen Feuchtesensor zum Messen der relativen Feuchte im Nutzraum;
   ein Temperiersystem, das ausgelegt ist, eine Temperatur im Nutzraum zu erhöhen;
   einen Temperatursensor zum Messen der Temperatur im Nutzraum;
   wobei das Befeuchtungssystem mindestens einen Wirkstoffanschluss umfasst, der zur Verbindung mit einem Wirkstoffbehälter, der eine Wirkstoffflüssigkeit mit einem Wirkstoff enthält, ausgelegt ist; und
   wobei das Gerät dazu ausgelegt ist, den Wirkstoff über das Befeuchtungssystem in den Nutzraum einzubringen.
G2. Gerät nach der vorstehenden Gerätausführungsformen, wobei das Befeuchtungssystem weiterhin mindestens einen Abfallanschluss umfasst, der zur Verbindung mit einem Abfallbehälter ausgelegt ist wobei das Gerät dazu ausgelegt ist, zumindest einen Teil des Befeuchtungssystems zu spülen und dabei anfallende Abfallflüssigkeit zu dem Abfallanschluss zu leiten.
G3. Gerät nach einer der vorstehenden Gerätausführungsformen, wobei das Befeuchtungssystem einen Befeuchter umfasst, der dazu ausgelegt ist Flüssigkeiten zu verdampfen und/oder zu vernebeln.
G4. Gerät nach der vorstehenden Gerätausführungsform, wobei der Befeuchter ein Verdampfer und/oder ein Vernebler ist.
G5. Gerät nach einer der vorstehenden Gerätausführungsformen, wobei das Befeuchtungssystem einen Wasseranschluss umfasst, der zur Verbindung mit einem Wasserreservoir, welches Frischwasser bereitstellt, ausgelegt ist.
G6. Gerät nach einer der vorstehenden Gerätausführungsformen, wobei das Befeuchtungssystem eine Pumpe umfasst.
G7. Gerät nach der vorstehenden Gerätausführungsform, wobei die Pumpe eine bidirektionale Pumpe ist.

Es versteht sich, dass eine bidirektionale Pumpe in beide Richtungen fördern kann.
G8. Gerät nach einer der 2 vorstehenden Gerätausführungsformen und mit den Merkmalen der Ausführungsform G3, wobei die Pumpe dazu ausgelegt ist Flüssigkeiten vom Wirkstoffanschluss zum Befeuchter zu fördern.
G9. Gerät nach einer der 3 vorstehenden Gerätausführungsformen und mit den Merkmalen der Ausführungsformen G3 und G5, wobei die Pumpe dazu ausgelegt ist Flüssigkeiten vom Wasseranschluss zum Befeuchter zu fördern.
G10. Gerät nach einer der vorstehenden Gerätausführungsformen wobei das Befeuchtungssystem eine Ventilmatrix umfasst, die dazu ausgelegt ist fluidische Verbindungen zwischen verschiedenen Anschlüssen zu ermöglichen.
G11. Gerät nach der vorstehenden Gerätausführungsform wobei die Ventilmatrix weiter dazu ausgelegt ist die Durchflussmenge für mindestens eine fluidische Verbindung einzustellen.
G12. Gerät nach einer der 2 vorstehenden Gerätausführungsformen und mit den Merkmalen der G6, wobei die Ventilmatrix zwischen der Pumpe und dem Wirkstoffanschluss angeordnet ist.
G13. Gerät nach einer der 3 vorstehenden Gerätausführungsformen und mit den Merkmalen der G5 und G6, wobei die Ventilmatrix zwischen der Pumpe und dem Wasseranschluss angeordnet ist.
G14. Gerät nach einer der 3 vorstehenden Gerätausführungsformen und mit den Merkmalen der G2 und G6, wobei die Ventilmatrix zwischen der Pumpe und dem Abfallanschluss angeordnet ist.
G15. Gerät nach einer der 5 vorstehenden Gerätausführungsformen, wobei die Ventilmatrix mindestens 2 Ventile umfasst.
G16. Gerät nach einer der vorstehenden Gerätausführungsformen, wobei das Gerät zusätzlich eine Luftverteilung umfasst, die dazu ausgelegt ist die Luft im Nutzraum umzuwälzen.
G17. Gerät nach der vorstehenden Gerätausführungsform, wobei die Luftverteilung einen Lüfter umfasst.
G18. Gerät nach einer der vorstehenden Gerätausführungsformen, wobei das Gerät weiter ein Belüftungssystem umfasst, das dazu ausgelegt ist, dem Nutzraum Zuluft zuzuführen.
G19. Gerät nach einer der vorstehenden Gerätausführungsformen, wobei das Gerät dazu ausgelegt ist das Verfahren nach einer der vorstehenden Verfahrensausführungsformen durchzuführen.
G20. Gerät nach einer der vorstehenden Gerätausführungsformen, wobei das Gerät eine Verarbeitungseinheit umfasst, die dazu ausgelegt ist das Gerät zu steuern.
G21. Gerät nach der vorstehenden Gerätausführungsform, wobei die Verarbeitungseinheit dazu ausgelegt ist das Verfahren nach einem der vorstehenden Verfahrensausführungsformen durchzuführen.
G22. Gerät nach einer der vorstehenden Gerätausführungsformen, wobei die Wirkstoffflüssigkeit eine Wirkstofflösung ist.
G23. Gerät nach einer der vorstehenden Gerätausführungsformen, wobei der Wirkstoff ein Sterilisationsmittel ist.
G24. Gerät nach einer der vorstehenden Gerätausführungsformen, wobei der Wirkstoff Wasserstoffperoxid (H₂O₂) ist.
G25. Gerät nach einer der vorstehenden Gerätausführungsformen, wobei das Gerät ein Laborgerät ist.
G26. Gerät nach einer der vorstehenden Gerätausführungsformen, wobei das Gerät ein Klima- oder Wärmegerät ist.
G27. Gerät nach einer der vorstehenden Gerätausführungsformen, wobei das Gerät ein Inkubator ist.
G28. Gerät nach einer der vorstehenden Gerätausführungsformen mit den Merkmalen der Ausführungsformen G3 und G6, wobei das Befeuchtungssystem weiter einen Flüssigkeitssensor umfasst, der zwischen dem Befeuchter und der Pumpe angeordnet ist.
V60. Verfahren nach einer der vorstehenden Verfahrensausführungsformen, wobei das Gerät ein Gerät nach einer der vorstehenden Gerätausführungsformen ist.

Nachfolgend wird auf Systemausführungsformen Bezug genommen. Diese Ausführungsformen sind durch ein S, gefolgt von einer Zahl gekennzeichnet. Wenn im Folgenden von Systemausführungsformen oder auch S-Ausführungsformen die Rede ist, sind diese Ausführungsformen gemeint.
S1. System umfassend
   Gerät nach einer der vorstehenden Gerätausführungsformen; und
   Abbaueinheit, die dazu ausgelegt ist, einen Wirkstoff im Nutzraum des Gerätes abzubauen.
S2. System nach der vorstehenden Systemausführungsform, wobei die Abbaueinheit dazu ausgelegt ist im Nutzraum platziert und elektrisch mit dem Gerät verbunden zu werden.
S3. System nach einer der vorstehenden Systemausführungsformen, wobei die Abbaueinheit eine UV-Lampe umfasst.
S4. System nach einer der vorstehenden Systemausführungsformen, wobei die Abbaueinheit einen Reaktionspartner und/oder Katalysator für den Wirkstoff umfasst.
S5. System nach der vorstehenden Systemausführungsform, wobei die Abbaueinheit einen Lüfter umfasst, der dazu ausgelegt ist Luft über den Reaktionspartner und/oder Katalysator zu leiten.

Ausführungsformen der vorliegenden Erfindung werden nun unter Bezugnahme auf die beigefügten Zeichnungen beschrieben. Diese Ausführungsformen sollen die vorliegende Erfindung nur beispielhaft darstellen, aber nicht einschränken.
- Fig. 1: zeigt ein Gerät mit Nutzraum, Befeuchtungssystem und Temperiersystem;
- Fig. 2: zeigt eine Ausführungsform eines Geräts gemäß der vorliegenden Erfindung;
- Fig. 3: zeigt eine Ausführungsform eines Verfahrens zur Reduzierung der Keimdichte;
- Fig. 4: zeigt eine weitere Ausführungsform eines Verfahrens zur Reduzierung der Keimdichte; und
- Fig. 5: illustriert schematisch einen Verlauf von Temperatur und relativer Feuchtigkeit während einer Umsetzung des Verfahrens zur Reduzierung der Keimdichte.

Es wird darauf hingewiesen, dass nicht alle Zeichnungen alle Bezugszeichen tragen. Stattdessen sind in einigen der Zeichnungen einige der Bezugszeichen der Kürze und Einfachheit der Darstellung halber weggelassen worden. Ausführungsformen der vorliegenden Erfindung werden nun unter Bezugnahme auf die beigefügten Zeichnungen beschrieben.

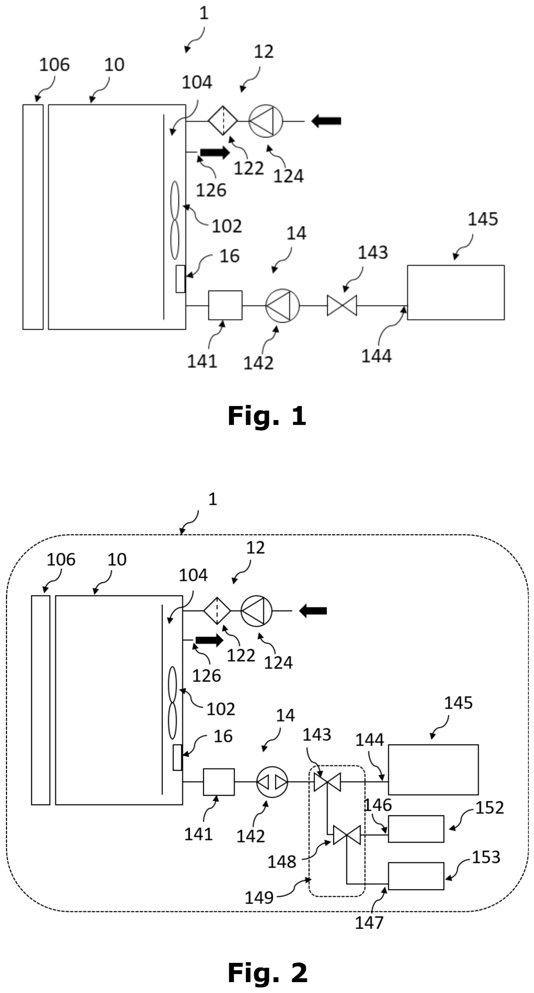

Fig. 1 zeigt ein Gerät 1 mit einem Nutzraum 10, einem Befeuchtungssystem 14 und einem Temperiersystem 16, die dazu ausgelegt sind die relative Feuchte bzw. die Temperatur im Nutzraum 10 zu erhöhen. Zusätzlich kann das Gerät 1 beispielsweise auch ein Belüftungssystem 12 aufweisen, welches dazu ausgelegt ist dem Nutzraum 10 Zuluft (z.B. Umgebungsluft) zuzuführen. Es versteht sich, dass das Gerät darüber hinaus auch weitere Komponenten umfassen kann, beispielsweise kann das Gerät 1 zusätzlich eine CO₂-Steuerung oder -Regelung aufweisen, die es ermöglicht die CO₂-Konzentration im Nutzraum zu erhöhen. Der Nutzraum kann allgemein über mindestens eine Tür 106 für einen Nutzer außerhalb des Geräts zugänglich sein.

Es versteht sich, dass die Erfindung grundsätzlich ein Gerät 1 betrifft, welches einen Nutzraum 10, ein aktives Befeuchtungssystem 14 und ein Temperiersystem 16 umfasst, wie bspw. allgemein ein Labor-, Klima- oder Wärmegerät. Insbesondere kann dies ein Inkubator sein.

Das Gerät 1 kann weiterhin eine Luftverteilung umfassen, die einen Lüfter (bzw. Ventilator) 102 aufweisen kann, der dazu ausgelegt ist, die Luft im Nutzraum 10 umzuwälzen. Der Lüfter 102 kann vorzugsweise in einem Luftkanal 104 angeordnet sein.

Das Temperiersystem 16 kann beispielsweise ein Temperierelement umfassen, welches in einem entsprechenden Luftkanal 104 angeordnet ist und die vorbeiströmende Luft temperiert, z.B. erwärmt oder auch kühlt. Alternativ kann das Temperierelement auch außen an einem Innenbehälter angebracht sein, der den Nutzraum definiert und so ein oder mehrere Wände des Nutzraums 10 erwärmen. Das Temeprierelement kann beispielsweise ein Heizelement (z.B. ein Widerstandsheizelement) oder auch ein Peltiersystem sein. Weiterhin kann das Gerät 1 einen Temperatursensor umfassen (nicht gezeigt), der das Messen der Temperatur im Nutzraum ermöglicht. Dadurch kann das Temperiersystem 16 entsprechend gesteuert oder geregelt werden. Es versteht sich, dass das Temperiersystem 16 auch mehrere Temperierelemente umfassen kann

Das Befeuchtungssystem 14 kann einen Befeuchter 141, eine Pumpe 142 und einen Wasseranschluss 144 umfassen. Der Befeuchter 141 kann insbesondere ein Vernebler oder Verdampfer umfassen, die flüssiges Wasser vernebeln, bzw. verdampfen und so die relative Feuchte der Luft im Nutzraum erhöhen können. Das Wasser kann entsprechend in einem Wasserreservoir 145 bereitgestellt werden, welches in manchen Ausführungsformen von dem Gerät umfasst wird und in anderen in diesem platziert oder extern zu diesem angeordnet ist.

Das Wasser aus dem Wasserreservoir 145 kann dann über den Wasseranschluss 144, an dem das Wasserreservoir 145 angeschlossen werden kann, mittels der Pumpe 142 zum Befeuchter 141 gefördert werden. Zusätzlich kann noch ein erstes Ventil 143 vorgesehen sein, welches vorzugsweise zwischen Pumpe 142 und Wasseranschluss 144 angeordnet ist und die fuldische Verbindung zwischen diesen gezielt unterbrechen kann.

Das Gerät kann außerdem einen Feuchtesensor (nicht gezeigt) zum Messen der relativen Feuchte im Nutzraum umfassen. Dies ermöglicht eine Steuerung oder Regelung der relativen Feuchte im Nutzraum mittels dem Befeuchtungssystem 14 in Abhängigkeit von der gemessenen relativen Feuchte.

Das optionale Belüftungssystem 12 kann beispielsweise eine (Luft-)Pumpe 124 umfassen, die Zuluft (z.B. Umgebungsluft) in den Nutzraum 10 fördern kann. Vorzugsweise kann das Belüftungssystem 12 außerdem einen Filter 122 umfassen, der die Zuluft filtert um Kontamination des Nutzraumes 10 zu vermeiden. Der Filter kann beispielsweise ein Membranfilter sein. Zusätzlich kann das Gerät eine Druckausgleichsöffnung 126 umfassen, die Teil des Belüftungssystems sein kann. Dadurch kann vorteilhaft verhindert werden, dass der Druck im Nutzraum bei der aktiven Zuführung von Zuluft zu stark ansteigt. Vorzugsweise weist die Druckausgleichsöffnung 126 einen Strömungswiderstand auf, der einen ständigen Luftaustausch mit der Umgebung des Geräts 1 erschwert bzw. reduziert, sodass ein entsprechender Luftaustausch vor allem bei einer Druckdifferenz zwischen Nutzraum und Umgebung stattfindet und ansonsten vorzugsweise stark gehemmt ist.

Es versteht sich, dass die Pumpe 124 alternativ auch Luft aus dem Nutzraum absaugen könnte und dadurch Zuluft durch die Druckausgleichsöffnung 126 einströmt. Insbesondere in solchen Ausführungsformen kann der Strömungswiderstand vorzugsweise durch einen Filter bereitgestellt werden.

Der vorliegenden Erfindung liegt die Idee zugrunde, das bereits vorhandene Befeuchtungssystem 14 in einem Gerät 1 für die Einbringung eines Wirkstoffs (vorzugsweise eines Sterilisationsmittels) in den Nutzraum 10 zu verwenden.

Mit Bezug auf Figur 2 kann vorgesehen sein, dass das Befeuchtungssystem 14 einen Wirkstoffanschluss 146 umfasst, der zur Verbindung mit einem Wirkstoffbehälter 152 ausgelegt ist. Dieser Wirkstoffbehälter 152 kann eine Wirkstoffflüssigkeit, vorzugsweise ein Sterilisationsmittel, weiter bevorzugt Wasserstoffperoxid, umfassen. Das Befeuchtungssystem kann auch mehr als einen Wirkstoffbehälteranschluss umfassen.

Der Wirkstoffanschluss 146 kann über mindestens ein erstes Ventil 143 mit der Pumpe 142 verbunden sein. Beispielsweise kann das erste Ventil 143 ein 3-Wege-Ventil sein, welches wahlweise den Wasseranschluss 144 oder den Wirkstoffanschluss 146 mit der Pumpe 142 verbinden kann.

Weiterhin kann das Befeuchtungssystem einen Abfallanschluss 147 umfassen, der zur Verbindung mit einem Abfallbehälter 153 ausgelegt ist. Der Abfallbehälter 153 kann dazu ausgelegt sein, Abfallflüssigkeiten aufzunehmen.

Der Abfallbehälter 153 kann ebenfalls über das erste Ventil 143 mit der Pumpe 142 verbunden sein. Vorzugsweise kann ein zweites Ventil 148 vorgesehen sein, welches beispielsweise wahlweise den Abfallanschluss 147 oder den Wirkstoffanschluss 146 mit dem ersten Ventil verbinden kann.

Allgemeiner kann eine Ventilmatrix 149 vorgesehen sein, die die Pumpe 142 wahlweise fluidisch mit dem Wasseranschluss 144, dem Wirkstoffanschluss 146 und dem Abfallanschluss 147 verbinden kann.

Vorzugsweise kann die Pumpe 142 bidirektional sein. Dadurch kann insbesondere eine effektive Spülung zumindest eines Teils der Zuleitungen ermöglicht werden.

Optional kann ein Flüssigkeitssensor zwischen der Pumpe 142 und dem Verdampfer 141 vorhanden sein, der sich möglichst nahe am Verdampfer 141 befindet und den Stand der Flüssigkeitssäule beim Spülen erfassen soll. Er soll Schwankungen der Fördermenge der 142 Pumpe ausgleichen und gewährleisten, dass ein Schlauch, der den Verdampfer 141 mit der Pumpe 142 verbindet, immer bis zu einer definierten Stelle mit Spülwasser gefüllt wird.

Insgesamt kann das Gerät somit auch dazu ausgelegt sein, gleichzeitig Flüssigkeiten aus mehr als einem Behälter und/oder Reservoir zu fördern. In anderen Worten kann das Gerät 1 und insbesondere das Befeuchtungssystem 14 so ausgelegt sein, dass gleichzeitig Flüssigkeiten über mehrere Anschlüsse von der Pumpe 142 gefördert werden können, beispielweise kann gleichzeitig Flüssigkeit von dem Wirkstoffanschluss 146 und dem Wasseranschluss 144 gefördert werden. Beispielsweise kann eine Wirkstoffflüssigkeit durch ein Vermischen von 2 oder mehr Flüssigkeiten hergestellt werden. Insbesondere kann aber auch eine konzentrierte Wirkstofflösung mit Wasser verdünnt werden. Dies kann vorteilhaft ermöglichen mehr Wirkstoff bei gleicher Wirkstoffbehältergröße bereitzustellen, oder bspw. dieselbe Menge Wirkstoff in einem kleineren Wirkstoffbehälter 152.

Allgemein kann die vorliegende Erfindung insbesondere ein System umfassen, welches neben dem Gerät 1 außerdem eine Abbaueinheit umfassen, die dazu ausgelegt ist, den Wirkstoff im Nutzraum 10 des Gerätes 1 abzubauen bzw. zu zersetzen. Die Abbaueinheit kann auch als Neutralisierungseinheit oder Zersetzungseinheit bezeichnet werden.

Die Abbaueinheit kann im Nutzraum 10 platziert werden und elektrisch mit dem Gerät 1 verbunden werden. Beispielsweise kann die Abbaueinheit eine UV-Lampe umfassen, welche mittels UV-Strahlung die Zersetzung des Wirkstoffs (z.B. Wasserstoffperoxid) bewirken kann, sodass nach erfolgter Reduktion der Keimdichte im Nutzraumes, eine sichere Atmosphäre im Nutzraum hergestellt werden kann, bevor ein Nutzer die Tür 106 zum Nutzraum 10 öffnet.

Vorzugsweise kann die Abbaueinheit einen Reaktionspartner oder Katalysator umfassen, der dazu ausgelegt ist den Wirkstoff abzubauen bzw. zu zersetzen. Weiter bevorzugt kann die Abbaueinheit einen Lüfter umfassen, der aktiv Luft über den Reaktionspartner bzw. Katalysator fördert um so die Effizienz zu erhöhen. Dadurch kann vorteilhaft schneller eine sichere Atmosphäre im Nutzraum bereitgestellt werden.

Das Gerät kann außerdem eine Verarbeitungseinheit umfassen, welche dazu ausgelegt sein kann, Daten an das Befeuchtungssystem 14, das Temperiersystem 16, die Luftverteilung und/oder das Belüftungssystem 12 zu senden (und ggf. auch zu von diesen zu empfangen), diese anzusteuern bzw. zu regeln und/oder diese zu kontrollieren. Insbesondere kann die Verarbeitungseinheit 15 mit dem Befeuchtungssystem 14 und dem Temperiersystem 16 verbunden sein. Entsprechende Verbindungen können kabelgebunden oder kabellos sein. Weiterhin kann die Verarbeitungseinheit 15 mit mindestens einem Sensor verbunden sein, der so angeordnet ist, dass dieser ein Signal indikativ für mindestens einen Parameter innerhalb des Nutzraums 10 bereitstellen kann. Der mindestens eine Sensor kann beispielsweise innerhalb des Nutzraums 10 angeordnet sein. Der mindestens eine Sensor kann insbesondere der Feuchtesensor und/oder der Temperatursensor sein.

Die Verarbeitungseinheit kann dazu ausgelegt sein mindestens eine Größe (bzw. einen Parameter) für die Bedingungen innerhalb des Nutzraums 10 zu regeln. Insbesondere kann die Verarbeitungseinheit dazu ausgelegt sein das Befeuchtungssystem 14 und das Temperiersystem so zu regeln, dass für mindestens einen Parameter ein vorgegebener Sollwert erreicht wird, insbesondere eine Temperatur und/oder relative Feuchte.

Die Verarbeitungseinheit kann außerdem dazu ausgelegt sein eine mit dem Gerät elektrisch verbundene Abbaueinheit zu steuern.

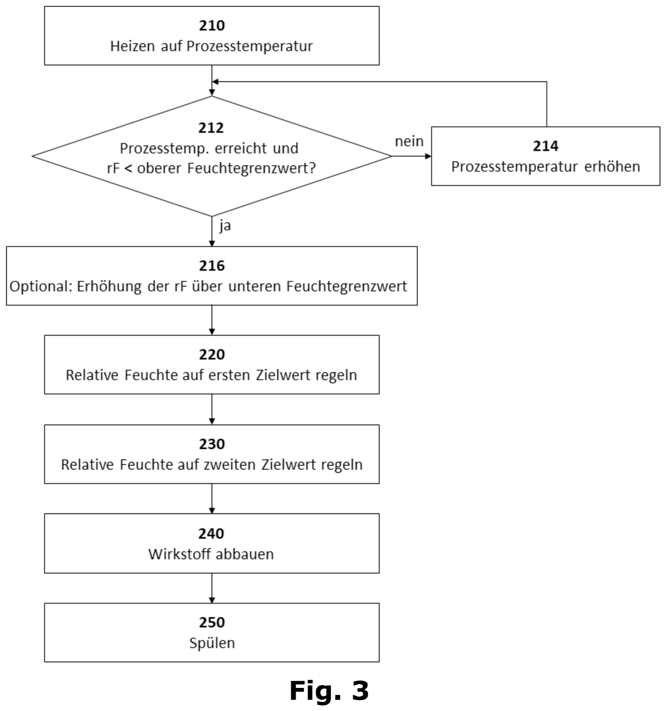

Die vorliegende Erfindung betrifft außerdem ein Verfahren zum Reduzieren der Keimdichte im Nutzraum eines Gerätes. Mit Bezug auf Fig. 3 umfasst das Verfahren das Aufheizen des Nutzraums auf eine Prozesstemperatur (Schritt 210). Die Prozesstemperatur kann vorzugsweise zunächst einem Initialwert entsprechen, der weiter bevorzugt einer vom Nutzer eingestellten Temperatur für nachfolgende Prozesse, z.B. einer Inkubationstemperatur, entsprechen kann und vorzugsweise mindestens 37°C beträgt. Zu diesem Zeitpunkt ist eine Regelung der Feuchte im Nutzraum ausgeschaltet, d.h. diese wird insbesondere nicht aktiv erhöht oder auf einem bestimmten Wert gehalten.

Nachdem die Prozesstemperatur (insbesondere der Initialwert) erreicht wurde (oder auch schon während dem Aufheizen) kann die relative Feuchte im Nutzraum bestimmt und mit einem oberen Feuchtegrenzwert verglichen werden (Schritt 212). Falls die relative Feuchte über dem oberen Feuchtegrenzwert liegt, wird die Prozesstemperatur erhöht um die relative Feuchte weiter abzusenken (Schritt 214). Die angepasste Prozesstemperatur kann dabei beispielsweise ausgehend von der aktuellen relativen Feuchte und Temperatur anhand der Dampfdruckkurve berechnet werden. Alternativ kann die Prozesstemperatur stufenweise erhöht werden und die relative Feuchte überprüft werden, bis der obere Feuchtegrenzwert unterschritten wird.

In Ausführungsformen der Erfindung kann vorgesehen sein, dass ein unterer Feuchtegrenzwert verwendet wird. Beispielsweise könnte die Prozesstemperatur 40 °C betragen, und bei dieser Temperatur könnte die relative Feuchte (in Schritt 212) 30 % betragen. Wenn der obere Feuchtegrenzwert beispielsweise 50 % beträgt, findet keine weitere Anpassung gemäß Schritt 214 statt. Zusätzlich kann vorgesehen sein, dass ein unterer Feuchtegrenzwert verwendet wird. Beispielsweise könnte dieser im Beispiel 40 % betragen. Wie beschrieben ist in dem Beispiel die relative Feuchte ohne weitere Maßnahmen bei 30 %. Ist diese unterhalb des unteren Feuchtegrenzwertes (im Beispiel 40 %), so kann die relative Feuchte im Schritt 216 erhöht werden, indem Wasser verdampft wird, bis die relative Feuchte über dem unteren Feuchtegrenzwert liegt. Die Verwendung eines unteren Feuchtegrenzwertes kann vorteilhafter dazu führen, dass der Prozess vergleichbarere Messwerte der rel. Feuchte zeigt und auch vergleichbarer in der biologischen Wirksamkeit wird.

Insgesamt wird der Nutzraum also entsprechend auf eine Prozesstemperatur aufgeheizt, sodass die relative Feuchte im Nutzraum unter dem oberen Feuchtegrenzwert liegt (Schritte 210, 212, 214). Optional kann vorgesehen sein, dass die relative Feuchte im Nutzraum über einem unterem Feuchtegrenzwert liegt (Schritt 216). Die finale Prozesstemperatur wird anschließend über den Verlauf des gesamten Verfahrens konstant gehalten. Die relative Feuchte, die wie beschrieben erreicht wird (über Einstellung der finalen Prozesstemperatur und optionalem Verdampfen von Wasser, falls ein unterer Feuchtegrenzwert verwendet wird), wird im Laufe des Verfahrens als Startfeuchte verwendet.

Das vorliegende Verfahren kann somit vorteilhaft bei 37 °C oder geringfügig höheren Temperaturen (z.B. 50°C) durchgeführt werden, sodass keine oder zumindest keine signifikante Abkühlzeit erforderlich ist bis das Gerät nach Abschluss des Verfahrens für andere Prozesse verwendet werden kann. Unter anderem dadurch kann das vorliegende Verfahren vorteilhaft schneller als eine typische 180°C Sterilisationsroutine durchgeführt werden. Ebenso sind die Anforderungen an die Materialien für das Gesamtsystem geringer, was die Materialauswahl erweitert, da dieses keiner 180°C-Beständigkeit bedarf. Ebenso kann vorteilhaft die Isolationsdicke des Gerätes im Vergleich zu einem Gerät, welches für eine entsprechende Hitze-Sterilisation ausgebildet ist, reduziert werden.

Die geringere Oberflächentemperatur innerhalb des Gerätes während der Durchführung des Verfahrens ermöglicht außerdem vorteilhaft Materialien zu behandeln, die nicht für Temperaturen im Bereich von 180°C ausgelegt sind und erweitert so die Sterilisationsmöglichkeiten. Ebenso kann ein entsprechendes Verfahren für eine Reinraumanwendung vorteilhaft sein, da das Risiko für eine vermehrte Freisetzung von Partikeln und Störung der Reinraumluftströmung durch thermische Effekte wesentlich geringer ist gegenüber einer Sterilisation bei 180°C.

Die Anpassung der Prozesstemperatur, sodass die relative Feuchte unter einem oberen Feuchtegrenzwert liegt, ermöglicht außerdem vorteilhaft den Prozess automatisch an Umgebungsbedingungen am Ausstellungsort anzupassen, da die Prozesstemperatur abhängig von der relativen Feuchte angepasst wird. Dadurch ist beispielsweise auch eine Anwendung in tropischen Gebieten mit einer hohen Luftfeuchtigkeit ohne spezielle Anpassungen des Verfahrens möglich.

Nachdem die Prozesstemperatur stabilisiert wurde und die relative Feuchte, d.h. die Startfeuchte, unter dem oberen Feuchtegrenzwert liegt (und optional über einem unteren Feuchtegrenzwert liegt), wird die relative Feuchte im Nutzraum für eine erste Dauer auf einen ersten Zielwert geregelt (Schritt 220). Dabei bringt das Befeuchtungssystem einen bereitgestellten Wirkstoff, vorzugsweise H₂O₂, in den Nutzraum ein. Insbesondere kann das Einbringen des Wirkstoffs ein Verdampfen oder Vernebeln (beispielsweise Zerstäuben) einer Wirkstoffflüssigkeit, vorzugsweise einer H₂O₂-Lösung, umfassen. Der erste Zielwert kann vorzugsweise einen ersten Betrag über der Startfeuchte, d.h. der aktuellen relativen Feuchte liegen. Vorzugsweise liegt dieser erste Betrag im Bereich von 10%-40% relativer Feuchte, weiter bevorzugt im Bereich von 15%-30% relativer Feuchte, noch weiter bevorzugt im Bereich von 15%-25% relativer Feuchte.

Durch die Verwendung der Sterilisationsflüssigkeit zur Erhöhung der relativen Feuchte wird ein darin enthaltener Wirkstoff (vorzugsweise H₂O₂) im Nutzraum freigesetzt. Dieser kann dann entsprechend relevante Oberflächen erreichen und die Keimdichte (und somit die Keimbelastung) reduzieren. Weiterhin kann ein entsprechender Lüfter bzw. eine Luftverteilung des Geräts die Luft im Nutzraum zirkulieren und so den Wirkstoff gleichmäßig verteilen. Hat die relative Feuchte den ersten Zielwert erreicht, wird diese für die erste Dauer konstant gehalten.

Optional kann die Wirkstoffflüssigkeit auch aus mindestens zwei verschiedenen Behältern und/oder Reservoirs gemischt werden. Insbesondere kann beispielsweise eine hochkonzentrierte Wirkstofflösung mit Wasser verdünnt werden. Hierzu können beispielsweise über ein oder mehrere Ventile, z.B. eine Ventilmatrix, mehrere Behälter und/oder Reservoire gleichzeitig mit der Pumpe verbunden sein, die diese Flüssigkeiten zu dem Befeuchter fördert.

Das vorliegende Verfahren benötigt vorteilhaft nur einen Sensor für die relative Feuchte im Nutzraum, der typischerweise bereits vorhanden ist, wenn ein Befeuchtungssystem vorgesehen ist. Es wird insbesondere kein Sensor benötigt, mit dem auch die Wirkstoffkonzentration erfasst wird. Das Verfahren kann vielmehr alleine über den Feuchtesensor geregelt und/oder gesteuert werden.

Nach Ablauf der ersten Dauer kann optional (bei Bedarf) der obige Vorgang wiederholt werden. Insbesondere kann die aktuelle relative Feuchte im Nutzraum bestimmt werden und anschließend die relative Feuchte für eine zweite Dauer auf einen zweiten Zielwert geregelt werden (Schritt 230). Dies kann vorteilhaft die Keimdichte weiter reduzieren und beispielweise eine Sterilisation der Oberflächen im Nutzraum ermöglichen.

Auch der zweite Zielwert kann vorzugsweise einen zweiten Betrag über der aktuellen relativen Feuchte liegen. Wie zuvor liegt dieser zweite Betrag vorzugsweise im Bereich von 10%-40% relativer Feuchte, weiter bevorzugt im Bereich von 15%-30% relativer Feuchte, noch weiter bevorzugt im Bereich von 15%-25% relativer Feuchte. Der erste Betrag und der zweite Betrag können vorzugsweise identisch sein.

Anschließend, d.h. nach Ablauf der ersten Dauer oder der zweiten Dauer, wird die Feuchteregelung deaktiviert und der Wirkstoff im Nutzraum abgebaut (Schritt 240). Vorzugsweise wird der Wirkstoff mittels einer Abbaueinheit abgebaut. Die Abbaueinheit kann zu Beginn des Verfahrens in den Nutzraum des Gerätes eingebracht werden und mit diesem elektrisch verbunden werden. Dies ermöglicht vorteilhaft, den Wirkstoff soweit abzubauen, dass eine für einen Nutzer ungefährliche Restkonzentration des gasförmigen Wirkstoffs erreicht wird. Hierzu kann die Abbaueinheit vorzugsweise zeitgesteuert sein.

Nachdem der Wirkstoff abgebaut wurde, können die Zuleitungen zu einem Befeuchter (z.B. Verdampfer) des Befeuchtungssystem zumindest teilweise in einen Abfallbehälter entleert und gespült werden, insbesondere bidirektional (Schritt 250). D.h. frisches Wasser kann von einem entsprechenden Reservoir mittels einer Pumpe zum Befeuchter gepumpt werden und anschließend in den Abfallbehälter. Dieser Vorgang kann mehrfach wiederholt werden um Rückstände der Wirkstoffflüssigkeit in den Zuleitungen des Befeuchters zu entfernen.

Schlussendlich können der Abfallbehälter und der Wirkstoffbehälter entsorgt werden und das Gerät wieder in den Normalbetrieb verwendet werden.

Die Verarbeitungseinheit des Gerätes kann vorzugsweise dazu ausgelegt sein, das Verfahren zur Reduzierung der Keimdichte durchzuführen.

Die vorliegende Erfindung nutzt somit vorteilhaft bereits vorhandene Bauteile von Geräten um die Reduktion der Keimdichte zu ermöglichen. Genauer kann ein vorhandenes Befeuchtungssystem vorteilhaft dahingehend erweitert werden, dass auch die Reduktion der Keimdichte und insbesondere eine Desinfektion und Sterilisation ermöglicht werden kann. Die Verwendung von Teilen des Befeuchtungssystems kann außerdem vorteilhaft ermöglichen, dass zumindest ein Teil des Leitungssystems, das im Normalbetrieb für Wasser verwendet wird, mit flüssigem Sterilisationsmittel dekontaminiert wird, bzw. die Keimdichte in den Teilen des Leitungssystems entsprechend reduziert wird

Insgesamt ermöglicht das vorliegende Verfahren vorteilhaft einen automatisierten und geregelten Prozess, der über das Gerät (z.B. den Inkubator) geregelt werden kann.

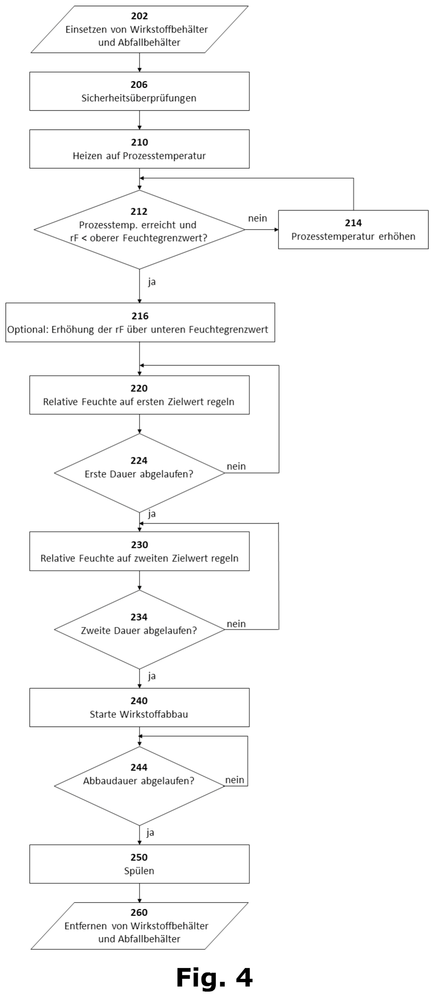

Fig. 4 zeigt ein etwas detaillierteres Ablaufschema einer beispielhaften Ausführungsform des Verfahrens zur Reduzierung der Keimdichte im Nutzraum eines Gerätes. Das Verfahren wird nachfolgend im Hinblick auf eine Sterilisation der Oberflächen im Nutzraum beschreiben, es versteht sich jedoch, dass auch lediglich eine Reduzierung der Keimdichte oder Desinfektion des Nutzraums erfolgen kann, d.h. nicht zwingend eine Sterilisation erfolgen muss.

Zu Beginn setzt der Nutzer einen Wirkstoffbehälter und einen Abfallbehälter in das Gerät ein (Schritt 202). In anderen Worten kann der Nutzer eine Kartusche mit flüssigem Wirkstoff (auch Sterilisationsmittel) einsetzen, bspw. Wasserstoffperoxid, und eine Abfallkartusche für Flüssigkeiten anschließen. Der Wirkstoffbehälter kann dabei typischerweise eine Lösung umfassen, die den Wirkstoff (vorzugsweise H₂O₂) und Wasser umfasst. Zusätzlich kann vorgesehen sein, dass der Nutzer eine Abbaueinheit (auch Zersetzungseinheit oder Neutralisierungseinheit genannt) in den Nutzraum des Gerätes einbringt und elektrisch mit diesem Gerät verbindet. Die Abbaueinheit kann grundsätzlich dazu ausgelegt sein, den Wirkstoff für einen Nutzer unschädlich zu machen (z.B. zu neutralisieren und/oder zu zersetzen). Beispielsweise kann die Abbaueinheit eine UV-Lampe umfassen oder aus dieser bestehen, oder alternativ einen Lüfter mit einem Reaktionspartner für den Wirkstoff oder einem Katalysator umfassen, um die Luft aktiv darüber zu fördern.

Anschließend kann vorgesehen sein, dass Sicherheitsüberprüfungen durchgeführt werden (Schritt 206). Zum Beispiel kann überprüft werden, dass der Nutzraum des Gerätes geschlossen und gegen unerlaubtes Öffnen gesichert ist und/oder dass die Abbaueinheit im Nutzraum platziert und angeschlossen wurde. Zusätzlich oder alternativ kann überprüft werden, ob andere Geräteöffnungen geschlossen sind (beispielsweise Durchführungsöffnungen, die für den Nutzer zugänglich sind), ob ein interner Gerätelüfter funktioniert und/oder ob ein optionaler Flüssigkeitssensor zwischen der Pumpe 142 und dem Verdampfer 141 funktioniert.

Anschließend kann damit begonnen werden, den Nutzraum für die Sterilisation zu konditionieren. In einem ersten Schritt wird hierzu der Nutzraum und insbesondere die Luft des Nutzraums bis zu einem Initialwert (d.h. bis zu einer initialen Prozesstemperatur) aufgeheizt (Schritt 210). Dieser beträgt mindestens 37°C und kann vom Nutzer festgelegt werden (z.B. kann die initiale Prozesstemperatur einer vom Nutzer gewählten Inkubationstemperatur entsprechen). Während dem Aufheizen ist die Feuchteregelung ausgeschaltet. Während des Aufheizens oder nach dem Erreichen der Prozesstemperatur kann die aktuelle Luftfeuchte (d.h. die relative Feuchte) im Nutzraum bestimmt und mit dem oberen Feuchtegrenzwert vergleichen werden (Schritt 212). Der obere Feuchtegrenzwert kann beispielsweise 55% relative Feuchte betragen. Wird der obere Feuchtegrenzwert unterschritten, bleibt die Prozesstemperatur wie initial eingestellt.

Falls die relative Feuchte nicht unter dem oberen Feuchtegrenzwert liegt, d.h. der obere Feuchtegrenzwert wird überschritten, wird die Prozesstemperatur entsprechend erhöht um die relative Feuchte im Nutzraum unter den oberen Feuchtegrenzwert zu reduzieren (Schritt 214). Die Prozesstemperatur kann dabei aus dem Messwert der aktuellen relativen Feuchte und Temperatur anhand der Dampfdruckkurve berechnet werden oder die Prozesstemperatur kann stufenweise erhöht werden und die relative Feuchte geprüft werden, bis der obere Feuchtegrenzwert unterschritten wird.

Die Reduktion der relativen Feuchte unter den entsprechenden oberen Feuchtegrenzwert dient dazu, sicherzustellen, dass ein erforderlicher Abstand zur Sättigungsgrenze vorhanden ist, da während des Einbringens des Wirkstoffes (z.B. durch Verdampfen einer Wirkstofflösung) die relative Feuchte im Nutzraum erhöht wird und eine Sättigung der Luft vermieden werden soll.

Auch in dieser Ausführungsform kann optional vorgesehen sein, dass ein unterer Feuchtegrenzwert verwendet wird, d.h., dass geprüft wird, dass die relative Feuchte oberhalb eines unteren Feuchtegrenzwertes liegt. Beispielsweise könnte dieser bei 30% oder 40% liegen, wobei im folgenden Beispiel ein unterer Feuchtegrenzwert von 40% angenommen wird. Wird in Schritt 210 beispielsweise eine Temperatur von 40 °C eingestellt und führt dies zu einer relativen Feuchte von 30 % (bei einem oberen Feuchtegrenzwert von 55% und einem unteren Feuchtegrenzwert von 40%), führt dies zu keiner weiteren Erhöhung der Prozesstemperatur gemäß Schritt 214. Allerdings kann die relative Feuchte gemäß Schritt 216 erhöht werden, indem Wasser verdampft wird. Die so erreichte Feuchte ist in diesem Beispiel die Startfeuchte.

Die relative Feuchte zum Start des Schrittes 220 (also nach Abschluss des womöglich mehrfachen durchlaufenen Schritt 212 und nach dem optionalen Schritt 216) ist die Startfeuchte und die entsprechend anliegende Prozesstemperatur wird für den Rest des Verfahrens konstant gehalten.

Anschließend kann die eigentliche Sterilisation (oder allgemeiner Reduzierung der Keimdichte) beginnen, indem relative Feuchte auf einen ersten Zielwert geregelt wird (Schritt 220). Hierzu wird die Flüssigkeit aus dem Wirkstoffbehälter (z.B. H₂O₂-Kartusche) zum Befeuchter gefördert, der diese entsprechend verdampft oder vernebelt und so den Wirkstoff in den Nutzraum einbringt. Der erste Zielwert kann dabei um einen (festen) ersten Betrag (z.B. 15%-25% relative Feuchte) über der aktuellen relativen Feuchte liegen.

Die Wirkstoffflüssigkeit (auch Sterilisationsflüssigkeit) wird verdampft (oder vernebelt) und dadurch erhöht sich die Luftfeuchte im Nutzraum. Der Wirkstoff wird dabei im Nutzraum freigesetzt und erreicht die zu sterilisierenden Oberflächen. Vorteilhaft kann der Wirkstoff durch eine Luftverteilung des Geräts gleichmäßig im Nutzraum verteilt werden.

Die relative Feuchte wird dann für eine erste Dauer auf dem ersten Zielwert gehalten (Schritt 224), d.h. die relative Feuchte wird konstant gehalten.

Nach Ablauf der ersten Dauer kann der Vorgang wiederholt werden um beispielsweise eine Sterilisierung bzw. eine log 12 Reduzierung sicherzustellen. Hierzu wird die aktuelle relative Feuchte bestimmt und anschließend auf einen zweiten Zielwert geregelt (Schritt 230). Der zweite Zielwert liegt dabei erneut einen (festen) zweiten Betrag (z.B. 15%-25% relative Feuchte) über der aktuellen relativen Feuchte. Dadurch steigt die Wirkstoffkonzentration im Nutzraum wieder an. Die relative Feuchte wird dann für eine zweite Dauer auf dem zweiten Zielwert gehalten (Schritt 234), d.h. die relative Feuchte wird konstant gehalten.

Nach Ablauf der zweiten Dauer kann die Feuchteregelung deaktiviert werden. Anschließend wird der Wirkstoffabbau im Nutzraum gestartet (Schritt 240). Hierzu kann vorzugsweise eine im Nutzraum eingebrachte Abbaueinheit von einer Gerätesteuerung für eine Abbaudauer (d.h. eine bestimmte Zeit) aktiviert werden (Schritt 244). Die Abbaudauer kann dabei vorzugsweise so gewählt werden, dass für den Nutzer keine Gefahr von der Restkonzentration des gasförmigen Wirkstoffs ausgeht, wenn dieser bspw. eine Tür zum Nutzraum öffnet.

Anschließend können entsprechende Flüssigkeitsleitungen zum Befeuchter (z.B. Verdampfer) gespült werden (Schritt 250). Hierzu können die Flüssigkeitsleitungen in den Abfallbehälter entleert und mit Frischwasser aus einem Wassertank gefüllt werden, welches anschließend wieder in den Abfallbehälter gepumpt werden kann. Dies kann mehrfach wiederholt werden.

Abschließend können Wirkstoffbehälter und Abfallbehälter entfernt und entsorgt werden (Schritt 260).

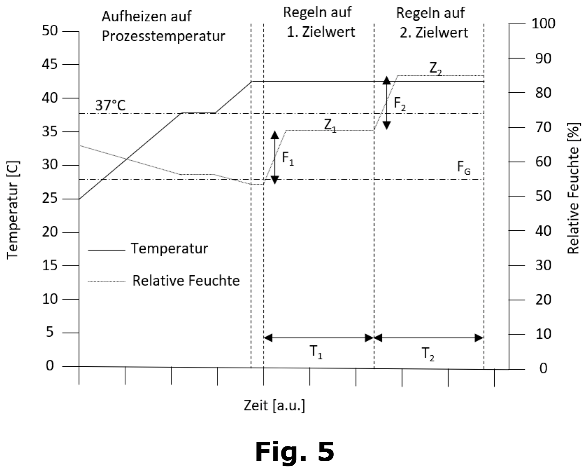

Fig. 5 illustriert einen beispielhaften Verlauf von Temperatur und relativer Feuchte im Nutzraum eines Gerätes bei Durchführung des Verfahrens zur Reduzierung der Keimdichte. Es versteht sich, dass dies eine rein beispielhafte und schematische Darstellung ist und Veränderungen von Temperatur und/oder relativer Feuchte beispielsweise typischerweise nicht rein linear verlaufen.

Zu Beginn ist die Luft im Nutzraum beispielsweise bei 25°C und ca. 65 % relativer Feuchte. Die Luft wird dann auf die Prozesstemperatur aufgeheizt. Zunächst wird die Temperatur hierzu auf einen Initialwert geheizt, der mindestens 37°C beträgt und im vorliegenden Beispiel diesem Wert entspricht. Durch das Erhöhen der Temperatur im Nutzraum sinkt die relative Feuchte. Im vorliegenden Beispiel wird nach dem Erreichen des Initialwertes, d.h. der 37°C, überprüft, ob die relative Feuchte unter einem entsprechenden oberen Feuchtegrenzwert F_{G} liegt, diese beträgt im vorliegenden Beispiel 55% relative Feuchte. Alternativ kann die relative Feuchte beispielsweise auch während dem Aufheizen mit dem oberen Grenzwert verglichen werden. Vorliegend liegt die relative Feuchte noch knapp über dem oberen Feuchtegrenzwert F_{G}. Daher wird die Prozesstemperatur angepasst und der Nutzraum entsprechend weiter aufgeheizt. Nach dem erneuten Aufheizen, auf ca. 43°C liegt die relative Feuchte unter dem oberen Feuchtegrenzwert F_{G}. Dementsprechend kann anschließend mit der eigentlichen Reduzierung der Keimbelastung begonnen werden. Hierzu wird die relative Feuchte für eine erste Dauer T₁ auf einen ersten Zielwert Z₁ geregelt, der beispielsweise einen ersten Betrag F₁ über der aktuellen Feuchte (auch Startfeuchte) liegt. Dabei wird mittels des Befeuchtungssystems des Gerätes eine Wirkstoffflüssigkeit, bevorzugt eine Wirkstofflösung verdampft. Dies ermöglicht die Einbringung von Wirkstoff in den Nutzraum.

Nach Ablauf der ersten Dauer T₁ kann die relative Feuchte bestimmt und erneut erhöht werden, bspw. um eine ausreichende Reduzierung der Keimdichte für eine Sterilisation sicherzustellen. In anderen Worten kann die relative Feuchte ausgehend von der dann aktuellen relativen Feuchte für eine zweite Dauer T₂ auf einen zweiten Zielwert Z₂ geregelt werden: Dieser zweite Zielwert Z₂ kann z.B. einen zweiten Betrag F₂ über der aktuellen relativen Feuchte liegen. Durch das Erhöhen der relativen Feuchte wird erneut Wirkstoff in den Nutzraum eingebracht.

Im vorliegenden Beispiel ist die erste Dauer T₁ identisch zu der zweiten Dauer T₂ und der erste Betrag F₁ identisch zum zweiten Betrag F₂. Diese können jedoch auch unterschiedlich zueinander gewählt werden.

Anschließend kann der Wirkstoff im Nutzraum abgebaut werden und Leitungen des Befeuchtungssystem gespült werden, wie vorstehend beschreiben. Dabei kann die Temperatur vorteilhaft weiterhin konstant gehalten werden um Kondensation innerhalb des Nutzraumes zu vermeiden. Aufgrund der Dichtheit des Gerätes fällt die Feuchtigkeit während des Wirkstoffabbaus typischerweise nicht ab.

Die vorliegende Erfindung ermöglicht somit bestehende Komponenten eines Befeuchtungssystems auch für die Reduzierung der Keimdichte im Nutzraum zu nutzen. Dabei können auch Teile des Leitungssystems vorteilhaft mit der verwendeten Wirkstoffflüssigkeit gereinigt werden. Ebenso kann auf hohe Temperaturen verzichtet werden, was zum einen die Materialauswahl für das Gerät erleichtert, die erforderliche Isolationsdicke reduziert und auch die Behandlungen von Gegenständen ermöglicht, die hohe Temperaturen von ca. 180°C nicht vertragen. Ebenso kann auf längere Abkühlphasen verzichtet werden, sodass das Gerät schneller wieder einsatzbereit sein kann.

Das Verfahren kann sich automatisch an Gegebenheiten des Aufstellungsortes anpassen (insbesondere an die relative Feuchte der Umgebung) und die Prozesstemperatur entsprechend anpassen. Insgesamt wird somit vorteilhaft eine automatische Durchführung des Verfahrens über eine geräteeigene Regelung ermöglicht.

Ein exemplarischer Prozess kann wie folgt ablaufen:
1. Ein Nutzer setzt eine Kartusche mit flüssigem Sterilisationsmittel ein (z.B. Wasserstoffperoxid) und schließt eine Abfallkartusche für Flüssigkeiten an.

Eine Abbaueinheit (beispielsweise eine Zersetzungseinheit) wird in den Nutzraum eingebracht und elektrisch mit dem Gerät verbunden, sie kann z.B. umfassen:
- einen Lüfter mit Reaktionspartner für Sterilisationsmittel oder mit Katalysator, um die Luft aktiv darüber zu fördern
- eine UV Lampe.

2.1 Start Konditionierungsphase: Aufheizen auf Sterilisationstemperatur (vom Nutzer eingestellte Inkubationstemperatur, beispielsweise mindestens 37°C), Regelung der Feuchte ist ausgeschaltet.

### 2.2 Kontrolle der Startfeuchte

Es ist verständlich, dass die Feuchte im Nutzraum während der Verdampfung in Schritt 3 erhöht wird. Vor dem Start dieser Verdampfung kann sicher gestellt werden, dass ein Abstand zur Sättigungsgrenze vorhanden ist. Der Verdampfungszyklus soll zweimalig durchlaufen werden. Vor diesem Hintergrund kann es vorteilhaft sein, die Startfeuchte relativ klein zu wählen.

Während des Aufheizens oder nach Erreichen der Sterilisationstemperatur wird die aktuelle Luftfeuchte im Nutzraum mit einem oberen Grenzwert verglichen - dieser könnte zum Beispiel 55% relativer Feuchte, also 55% rH betragen.

Wird der obere Grenzwert überschritten, wird im vorliegenden Fall, die Temperatur erhöht. Dadurch verringert sich der Wert der relativen Luftfeuchte im Nutzraum. Es kann eine neue Sterilisationstemperatur ermittelt werden. Mögliche Varianten, um die neue Sterilisationstempeatur zu ermitteln, sind:
- Zieltemperatur aus Messwert der aktuellen relativen Feuchte und Temperatur anhand der Dampfdruckkurve berechnen oder
- stufenweise den Temperaturwert des Gerätes erhöhen und die relative Feuchte wieder abprüfen, bis der Grenzwert der Startfeuchte unterschritten ist.

Wird der obere Grenzwert unterschritten, bleibt die Sterilisationstemperatur wie unter 2.1.

Optional kann auch ein unterer Feuchtegrenzwert verwendet werden. Dieser könnte beispielsweise bei 40% rH liegen. Liegt der Feuchtewert unterhalb des unteren Feuchtegrenzwertes, kann die relative Feuchte erhöht werden, beispielsweise, indem Wasser verdampft wird, bis der untere Feuchtegrenzwert überschritten wird.

Die Startfeuchte wird also durch Verwendung eines oberen Feuchtegrenzwert (und Erhöhung der Temperatur, solange dieser überschritten wird) und optional durch Verwendung eines unteren Feuchtegrenzwerts (und Verdampfen von Wasser, solange dieser unterschritten wird) erreicht. Die hierüber erzielte Feuchte ist die Startfeuchte.

Die wie beschriebene ermittelte Temperatur (Sterilisationstemperatur) wird über den gesamten weiteren Prozess vom Inkubator konstant gehalten.

### 3. Beginn Sterilisationsprozess

Die Regelung der Feuchte wird aktiviert, und Flüssigkeit wird aus der H₂O₂-Kartusche gefördert. Die H₂O₂-Kartusche kann auch als zweiter Behälter bezeichnet werden (wobei ein erster Behälter Wasser beinhaltet). Der Sollwert der relativen Luftfeuchte kann um einen festen Betrag (Z.B. 15-25%rF) über der aktuellen relativen Luftfeuchte liegen.

Die Sterilisationsflüssigkeit wird verdampft und dadurch erhöht sich die Luftfeuchte im Nutzraum. Der Wirkstoff wird dabei im Nutzraum freigesetzt und erreicht die zu sterilisierenden Oberflächen. Durch die normale Gerätelüftung wird das Mittel gleichmäßig im Nutzraum verteilt. Diese Feuchte wird konstant gehalten.

Nach Ablauf einer festgelegten Zeit wird der Sterilisationsvorgang wiederholt, indem die aktuelle relative Luftfeuchte nochmals gemessen und der Sollwert um einen festen Betrag (Z.B. 15-25%rF) erhöht wird. Dadurch steigt die Wirkstoffkonzentration im Nutzraum wieder an.

Diese Feuchte wird wieder eine festgelegte Zeit konstant gehalten.

### 4. Abbau des Sterilisationsmittels

Nach Ablauf einer Sterilisationszeit wird die Feuchteregelung deaktiviert. Die in den Nutzraum eingestellte Zersetzungseinheit wird von der Gerätesteuerung für eine bestimmte Zeit aktiviert.

Die Zeit wird so gewählt, dass keine Gefahr von der Restkonzentration gasförmigen H₂O₂ für den Anwender ausgeht.

5. Die Flüssigkeitsleitungen zum Verdampfer werden entleert (in einen Abfallbehälter) und mit Frischwasser aus dem Wassertank gefüllt und dieses wieder in den Abfallbehälter gepumpt. Dies kann mehrfach wiederholt werden.

6. Die Kartusche mit übrigem Sterilisationsmittel und Abfallkartusche werden entsorgt.

Wenn in dieser Beschreibung oder den Ansprüchen ein relativer Begriff wie "ungefähr", "im Wesentlichen" oder "annähernd" verwendet wird, sollte ein solcher Begriff auch so ausgelegt werden, dass er den exakten Begriff einschließt. Das heißt, z. B. "im Wesentlichen gerade" sollte so ausgelegt werden, dass er auch "(genau) gerade" einschließt.

Wann immer in den obigen oder auch in den beigefügten Ansprüchen Schritte rezitiert wurden, ist zu beachten, dass die Reihenfolge, in der die Schritte in diesem Text rezitiert werden, zufällig sein kann. Das heißt, dass die Reihenfolge, in der die Schritte rezitiert werden, zufällig sein kann, es sei denn, es wird etwas anderes angegeben oder es ist für den Fachmann klar. Das heißt, wenn im vorliegenden Dokument z. B. angegeben wird, dass ein Verfahren die Schritte (A) und (B) umfasst, bedeutet dies nicht notwendigerweise, dass Schritt (A) vor Schritt (B) erfolgt, sondern es ist auch möglich, dass Schritt (A) (zumindest teilweise) gleichzeitig mit Schritt (B) ausgeführt wird oder dass Schritt (B) vor Schritt (A) erfolgt. Wenn außerdem gesagt wird, dass ein Schritt (X) einem anderen Schritt (Z) vorausgeht, bedeutet dies nicht, dass es keinen Schritt zwischen den Schritten (X) und (Z) gibt. Das heißt, Schritt (X) vor Schritt (Z) umfasst die Situation, dass Schritt (X) direkt vor Schritt (Z) ausgeführt wird, aber auch die Situation, dass (X) vor einem oder mehreren Schritten (Y1), ..., gefolgt von Schritt (Z), ausgeführt wird. Entsprechende Überlegungen gelten, wenn Begriffe wie "nach" oder "vor" verwendet werden.

Während im Vorstehenden eine bevorzugte Ausführungsform unter Bezugnahme auf die begleitenden Zeichnungen beschrieben wurde, wird der Fachmann verstehen, dass diese Ausführungsform nur zu Veranschaulichungszwecken bereitgestellt wurde und keinesfalls als Einschränkung des Umfangs der vorliegenden Erfindung, die durch die Ansprüche definiert ist, ausgelegt werden sollte.

## Patentansprüche

1. Verfahren zum Reduzieren einer Keimdichte im Nutzraumes eines Geräts, umfassend
Aufheizen des Nutzraums auf eine Prozesstemperatur, sodass eine relative Feuchte im Nutzraum unter einem oberen Feuchtegrenzwert liegt;
Regelung der relativen Feuchte im Nutzraum auf einen ersten Zielwert für eine erste Dauer, wobei ein Befeuchtungssystem einen Wirkstoff einbringt;
Deaktivieren der Regelung der relativen Feuchte; und
Abbauen des Wirkstoffs im Nutzraum für eine Abbaudauer.

2. Verfahren nach Anspruch 1, wobei das Aufheizen des Nutzraumes auf die Prozesstemperatur ein Erhöhen der Prozesstemperatur umfasst, bis die relative Feuchte bei der Prozesstemperatur unter dem oberen Feuchtegrenzwert liegt.

3. Verfahren nach einem der vorstehenden Ansprüche, wobei die relative Feuchte während dem Aufheizen nicht geregelt wird.

4. Verfahren nach einem der vorstehenden Ansprüche, wobei die Prozesstemperatur nach dem Aufheizen mindestens bis zum Abbauen des Wirkstoffs im Nutzraum konstant gehalten wird.

5. Verfahren nach einem der vorstehenden Ansprüche, wobei das Verfahren weiter umfasst:
Erhöhung der relativen Feuchte im Nutzraum auf einen Wert oberhalb eines unteren Feuchtegrenzwertes.

6. Verfahren nach einem der vorstehenden Ansprüche, wobei der erste Zielwert einen ersten Betrag über der Feuchte zu Beginn der Regelung der relativen Feuchte auf einen ersten Zielwert liegt.

7. Verfahren nach einem der vorstehenden Ansprüche, wobei das Einbringen des Wirkstoffs das Verdampfen oder Vernebeln einer Wirkstoffflüssigkeit im Befeuchter des Befeuchtungssystems umfasst.

8. Verfahren nach einem der vorstehenden Ansprüche, wobei der Wirkstoff Wasserstoffperoxid (H₂O₂) ist.

9. Verfahren nach einem der vorstehenden Ansprüche, wobei das Verfahren weiterhin umfasst:
nach Ablauf der ersten Dauer,
Bestimmen der aktuellen relativen Feuchte im Nutzraum und
Regelung der relativen Feuchte auf einen zweiten Zielwert für eine zweite Dauer, wobei das Befeuchtungssystem eine Wirkstoffflüssigkeit einbringt, wobei der zweite Zielwert einen zweiten Betrag über der aktuellen relativen Feuchte liegt.

10. Verfahren nach einem der vorstehenden Ansprüche, wobei das Verfahren weiterhin umfasst: Verriegeln einer Tür zum Nutzraum des Geräts, wobei das Verriegeln vor dem Aufheizen des Nutzraums auf eine Prozesstemperatur durchgeführt wird, und wobei das Verfahren weiterhin umfasst: Durchführen eines automatischen Checks des Geräts, wobei der Check vor dem Aufheizen des Nutzraums auf eine Prozesstemperatur durchgeführt wird und das Aufheizen des Nutzraums auf eine Prozesstemperatur nur durchgeführt wird, wenn der Check erfolgreich ist.

11. Verfahren nach einem der vorstehenden Ansprüche, wobei das Gerät ein Klima- oder Wärmegerät ist oder ein Inkubator ist.

12. Gerät umfassend
einen Nutzraum;
ein aktives Befeuchtungssystem, das ausgelegt ist, eine relative Luftfeuchte im Nutzraum zu erhöhen;
einen Feuchtesensor zum Messen der relativen Feuchte im Nutzraum;
ein Temperiersystem, das ausgelegt ist, eine Temperatur im Nutzraum zu erhöhen; einen Temperatursensor zum Messen der Temperatur im Nutzraum;
wobei das Befeuchtungssystem mindestens einen Wirkstoffanschluss umfasst, der zur Verbindung mit einem Wirkstoffbehälter, der eine Wirkstoffflüssigkeit mit einem Wirkstoff enthält, ausgelegt ist; und
wobei das Gerät dazu ausgelegt ist, den Wirkstoff über das Befeuchtungssystem in den Nutzraum einzubringen.

13. Gerät nach Anspruch 12, wobei das Gerät dazu ausgelegt ist das Verfahren nach einem der Ansprüche 1 bis 11 durchzuführen.

14. System umfassend
Gerät nach einem der Ansprüche 12 und 13; und
Abbaueinheit, die dazu ausgelegt ist, einen Wirkstoff im Nutzraum des Gerätes abzubauen.
